# FASCICULE DE BREVET EUROPEEN

(11) **EP 2 978 503 B1**
(45) Date de publication et mention de la délivrance du brevet: **07.08.2019**
(21) Numéro de dépôt: 14718688.6
(22) Date de dépôt: 27.03.2014
(51) Int. Cl.: A61Q 19/00, A61Q 19/08, A61K 8/97, A61K 8/34, A61Q 5/00

(54) **UTILISATION COSMETIQUE D'UN EXTRAIT DE POLYGONUM BISTORTA**
KOSMETISCHE VERWENDUNG VON EINEM POLYGONUM BISTORTA EXTRAKT
COSMETIC USE OF AN EXTRACT OF POLYGONUM BISTORTA

(30) Priorité: 27.03.2013 FR 1352769
(43) Date de publication de la demande: 03.02.2016
(73) Titulaire: BASF Beauty Care Solutions France S.A.S., 69007 Lyon (FR); Université Claude Bernard Lyon I, 69622 Villeurbanne Cedex (FR); Centre National de la Recherche Scientifique (C.N.R.S.), 75016 Paris (FR)
(72) Inventeur: ANDRE-FREI, Valérie, F-69420 Ampuis (FR); BECHETOILLE, Nicolas, F-69530 Brignais (FR); PAIN, Sabine, F-38200 Vienne (FR); ROUSSELLE, Patricia, F-69005 Lyon (FR)
(74) Mandataire: Mendelsohn, Isabelle M. N.
(86) Numéro de dépôt international: PCT/FR2014/050731
(87) Numéro de publication internationale: WO 2014/155012

(56) Documents cités:
- FR-A1- 2 844 714
- FR-A1- 2 867 977
- US-A1- 2003 118 617
- DATABASE WPI Week 198529 Thomson Scientific, London, GB; AN 1985-174935 XP002716984, & JP S60 104005 A (KOBAYASHI KOSE KK) 8 juin 1985 (1985-06-08)
- DATABASE WPI Week 199803 Thomson Scientific, London, GB; AN 1998-029122 XP002716985, & JP H09 287982 A (NIPPONDENSO CO LTD) 4 novembre 1997 (1997-11-04)
- DATABASE GNPD [Online] MINTEL; 1 février 2012 (2012-02-01), "Multi Perfection Creme SPF 20", XP002716986, Database accession no. 1735662
- DATABASE GNPD MINTEL; 1 mai 2009 (2009-05-01), "Scalp Treatment Shampoo", XP002716987, Database accession no. 1129220

## Description

L'invention concerne l'utilisation cosmétique ou dermatologique par voie topique d'un extrait de Polygonum bistorta, pour stimuler l'expression du perlécane et/ou du dystroglycane, en particulier dans la matrice extracellulaire et/ou dans la membrane basale épithéliale, notamment la jonction dermo-épidermique.

Parmi les protéoglycannes à héparane sulfate (HSPGs) des lames basales, le perlécane présente un rôle majeur dans la morphogénèse de l'épithélium, notamment l'épiderme mais également dans la survie, la prolifération et la différenciation des kératinocytes et des cellules endothéliales, notamment cutanées. Celui-ci régule ces processus en contrôlant la biodisponibilité des facteurs de croissance.
Le dystroglycane est une glycoprotéine récepteur potentiel du perlécane. Le perlécane et le dystroglycane sont exprimés sur les membranes basales, structures ubiquitaires localisées au niveau de différents tissues tels que les épithéliums et endothéliums mais également au niveau de différents types cellulaires tels que les kératinocytes, fibroblastes, et cellules endothéliales. Ils interagissent ensemble et contribuent à assurer la solidité et la stabilité de la structure de la peau, des muqueuses et du cuir chevelu, en particulier l'épithélium, et notamment l'épiderme et le derme. Or au cours du vieillissement notamment chronobiologique, l'expression du perlécane et du dystroglycane est diminuée de façon drastique. En particulier, l'expression du perlécane diminue fortement au niveau de la jonction dermo-épidermique et des capillaires dermiques avec l'âge. Cette diminution n'est pas due à une dégradation de la protéine mais à une diminution de son expression, et tout particulièrement de sa régulation transcriptionnelle, par les kératinocytes et les cellules endothéliales. Par ailleurs, les inventeurs ont constaté qu'il existe une corrélation entre le défaut de synthèse du perlécane et l'épaisseur de la peau et/ou des muqueuses et/ou du cuir chevelu, en particulier de l'épithélium, préférentiellement l'épiderme. L'expression du perlécane et du dystroglycane est donc d'une grande importance dans l'homéostasie de la peau et/ou des muqueuses et/ou du cuir chevelu et dans le maintien de leur fermeté et de leur densité, qui sont dégradés lors du vieillissement notamment chronobiologique.

De façon surprenante les inventeurs ont découvert qu'un extrait, de Polygonum bistorta stimule l'expression des HSPGs et glycoprotéines des lames basales, et plus particulièrement du perlécane et du dystroglycane, en particulier dans la matrice extracellulaire et/ou dans la membrane basale épithéliale, notamment la jonction dermo-épidermique, plus particulièrement dans les kératinocytes et/ou les cellules endothéliales de la peau et/ou des muqueuses et/ou du cuir chevelu, et préférentiellement cutanées. Le fait de cibler à la fois les kératinocytes et les cellules endothéliales notamment cutanées permet d'avoir un double effet : 1) sur l'amélioration de la voie microvasculaire pour nourrir la peau, les muqueuses et/ou le cuir chevelu et 2) sur l'amélioration du teint en terme notamment de luminosité. Le fait de cibler non seulement le perlécane mais aussi le dystroglycane permet en outre d'agir de manière complète sur la voie d'expression et d'activité du perlécane.

Le Polygonum bistorta est une plante qui se trouve en Europe sauf en région méditerranéenne, Asie, Amérique du Nord jusqu'à une altitude de 2400m. Elle pousse dans les prairies humides et montagneuses, au bord des ruisseaux, sur les sols riches en azote. Elle est connue pour ses effets analgésiques par voie péritonéale, comme sédatif et astringent, et est décrit comme tonique puissant, cicatrisant et anti-diarrhéique.
La demande FR2867977 décrit des compositions cosmétiques, pharmaceutiques et dermo-cosmétiques destinées à prévenir et/ou lutter contre la formation des ridules et des rides de la peau en limitant les contractions des muscles sous-cutanés contenant du resvératrol et/ou certains de ses dérivés. Parmi les nombreux extraits végétaux pouvant contenir du resvératrol et/ou ces dérivés, le Polygonum bistorta est cité. Néanmoins, l'effet décrit dans ce brevet est un effet mécanique dû à une action myorelaxante en alternative à l'utilisation de toxine botulique. Il s'agit donc d'un mécanisme d'action et d'un résultat très différents de la voie d'action sur l'expression du perlécane et du dystroglycane selon l'invention qui se traduit par une augmentation de la fermeté et de la densité, effets opposés au relâchement cutané observé avec un myo-inhibiteur En outre, il est à noter que le dystroglycane contribue également à la contraction musculaire, ainsi l'augmentation de son expression induit donc un effet inverse à une myo-inhibition.
La présente invention concerne donc l'utilisation cosmétique ou dermatologique par voie topique d'un extrait de Polygonum bistorta, pour stimuler l'expression du perlécane et/ou du dystroglycane, en particulier dans la matrice extracellulaire et/ou dans la membrane basale épithéliale, notamment la jonction dermo-épidermique.
De manière préférentielle, l'utilisation selon l'invention est cosmétique et en application topique sur au moins une zone concernée de la peau saine et/ou d'une muqueuse saine et/ou du cuir chevelu sain, en particulier d'un être humain.
L'extrait de Polygonum bistorta est obtenu à partir de la racine par extraction aqueuse.

De manière préférentielle, l'utilisation de Polygonum bistorta stimule l'expression du perlécane et/ou du dystroglycane dans les kératinocytes et/ou les cellules endothéliales de la peau et/ou des muqueuses et/ou du cuir chevelu, préférentiellement les cellules endothéliales de la peau.

L'extrait végétal selon l'invention est extrait des racines.
L'extrait peut alors être obtenu par les méthodes d'extraction de végétaux connues dans le domaine, par exemple par macération d'au moins une partie de la plante de préférence entre 1 et 10% (p/p) par extraction aqueuse.

Au sens de la présente invention on entend par « extrait de Polygonum bistorta obtenu par extraction aqueuse» tout extrait obtenu par extraction avec une solution aqueuse, en particulier par macération dans une solution aqueuse, contenant plus de 60 % en poids, avantageusement au moins 70% en poids, en particulier au moins 80% en poids, plus particulièrement au moins 90% en poids, de façon particulière au moins 95% en poids, d'eau par rapport au poids total de la solution aqueuse, encore plus avantageusement ne contenant pas de butylène glycol, en particulier ne contenant pas d'alcool, plus particulièrement ne contenant que de l'eau.
Selon un mode de réalisation avantageux, l'extrait selon l'invention est obtenu par macération à froid, préférentiellement à 4°C, ou à température ambiante, c'est-à-dire entre 18 et 25°C, préférentiellement 20°C, éventuellement après une étape de séchage de la plante. Selon un mode préféré, l'extrait selon l'invention est obtenu par macération à température ambiante, préférentiellement 20°C.
Selon un mode de réalisation préférentielle, l'extrait selon l'invention est obtenu par macération pendant une durée entre 30 minutes et 24 heures, préférentiellement entre 1 heure et 5 heures, encore préférentiellement 2 heures.
L'extrait obtenu est ensuite de préférence centrifugé et/ou filtré et/ou distillé afin de récupérer la fraction soluble active. Il est préférentiellement filtré à un seuil de coupure de 0,45µm, encore préférentiellement 0,22 µm. Des étapes supplémentaires de décoloration et/ou désodorisation peuvent être effectuées sur l'extrait à n'importe quel stade de l'extraction et selon les techniques connues par l'Homme du métier.
L'extrait selon l'invention peut également être ensuite concentré par évaporation du solvant, par exemple par lyophilisation ou par atomisation. De façon particulièrement avantageuse, la quantité de plante lors de l'extraction de la racine, est de 1 % en poids par rapport au poids total du mélange plante/solvant d'extraction, préférentiellement solution aqueuse. En particulier la plante est broyée avant l'extraction. De façon avantageuse la durée de l'extraction est de 2 heures et effectuée à température ambiante, préférentiellement 20°C.

L'extrait selon l'invention ne contient pas de resvératrol et/ ou transstilbène (C₁₄H₁₂, MW 180,24 g/mol) et/ou de dérivés de resvératrol notamment sous forme d'oligomères de resvératrol et/ou d'analogues du resvératrol tel que la rhapontine (de formule C₂₁H₂₄O₉, MW 420,14 g/mol), la déoxyrhapontine (C₂₁H₂₄O₈,MW 404,14 g/mol), l'epsilone-viniférine, les acétates, les glucosides du resvératrol et le 3,4',5-trihydroxystilbène-3-O-beta-D-glucopyranoside (C₂₀H₂₂O₈, MW 390,13 g/mol).

L'extrait obtenu est préférentiellement soluble dans un solvant notamment polaire, tel que l'eau, un alcool, un polyol, un glycol, ou un de leurs mélanges, préférentiellement un mélange hydroglycolique, encore préférentiellement contenant un glycol choisi parmi le caprylyl glycol, l'hexylène glycol et leurs mélanges.

Avantageusement l'extrait est soluble dans une solution aqueuse contenant de l'hexylène glycol, en particulier contenant entre 0,1 et 10 % en poids d'hexylène glycol par rapport au poids total la solution aqueuse, plus particulièrement entre 1 et 5 % en poids d'hexylène glycol par rapport au poids total la solution aqueuse. De façon avantageuse l'extrait est soluble dans une solution aqueuse contenant du caprylyl glycol, en particulier contenant entre 0,01 et 5 % en poids de caprylyl glycol par rapport au poids total la solution aqueuse, plus particulièrement entre 0,1 et 1 % en poids de caprylyl glycol par rapport au poids total la solution aqueuse. Avantageusement la solution aqueuse ne contient pas de butylène glycol.

Au sens de la présente invention on entend par «peau saine», «muqueuse saine» ou «cuir chevelu sain», une zone de peau, muqueuse ou cuir chevelu sur laquelle est appliquée l'extrait selon l'invention et dite « non pathologique » par un dermatologue, c'est à dire ne présentant pas d'infection, de maladie ou d'affection cutanée telle que candidose, impétigo, psoriasis, eczéma, acné ou dermatite, ou de plaies ou de blessures.
De manière préférentielle, on entend au sens de la présente invention par « cosmétique » l'utilisation non-thérapeutique c'est à dire l'utilisation sur peau saine, muqueuse saine et/ou un cuir chevelu sain.
Au sens de la présente invention, on entend par «voie topique», l'application de l'extrait de Polygonum bistorta et/ou de la composition cosmétique contenant un tel extrait sur la surface de la zone concernée de la peau et/ou des muqueuses et/ou du cuir chevelu, en particulier d'un être humain, notamment par application directe ou par vaporisation.
Au sens de la présente invention, on entend par « stimulation de l'expression du perlécane et/ou du dystroglycane », une augmentation de l'expression génique et/ou protéique respectivement du perlécane et/ou du dystroglycane, préférentiellement de l'expression protéique. Cette augmentation peut être mesurée sur un modèle, comprenant au moins un type cellulaire présentant une expression du perlécane et/ou du dystroglycane, avantageusement les kératinocytes et/ou les cellules endothéliales, préférentiellement cutanées, au contact de l'extrait de Polygonum bistorta selon l'invention et se traduit par une augmentation de l'expression génique et/ou protéique respective du perlécane et/ou du dystroglycane égale ou supérieure à 10%, avantageusement égale ou supérieure à 20%, par rapport au niveau d'expression génique et/ou protéique dans un modèle témoin ou contrôle, c'est-à-dire sans mise au contact de l'extrait de Polygonum bistorta selon l'invention. L'augmentation de cette expression est préférentiellement protéique. De tels modèles sont décrits dans les exemples.
Selon l'invention, on désigne par les muqueuses notamment la muqueuse buccale, notamment buccale labiale, nasale, oculaire, anale et/ou urogénitale, en particulier labiale.

Avantageusement l'utilisation selon la présente invention est pour prévenir et/ou lutter contre le vieillissement de la peau et/ou des muqueuses et/ou du cuir chevelu notamment chronobiologique et/ou photobiologique, pour prévenir et/ou lutter contre la diminution de l'homéostasie de la peau et/ou des muqueuses et/ou du cuir chevelu et/ou pour l'améliorer, en particulier dans l'épiderme, pour renforcer la membrane basale épithéliale de la peau et/ou des muqueuses et/ou du cuir chevelu, préférentiellement la jonction dermo-épidermique, pour améliorer la prolifération et/ou la différenciation des kératinocytes, notamment au niveau épidermique, en particulier lié au vieillissement de la peau et/ou des muqueuses et/ou du cuir chevelu, pour prévenir et/ou lutter contre une diminution de la vascularisation de la peau et/ou des muqueuses et/ou du cuir chevelu et/ou pour l'améliorer, en particulier pour améliorer la structure des capillaires de la peau et/ou des muqueuses et/ou du cuir chevelu notamment cutanés, en particulier en augmentant l'expression de claudin 5 de la peau, pour améliorer la morphogenèse de l'épithélium, de la peau et/ou des muqueuses et/ou du cuir chevelu préférentiellement l'épiderme, pour restaurer l'architecture épithéliale de la peau et/ou des muqueuses et/ou du cuir chevelu, préférentiellement épidermique, en particulier des peaux et/ou des muqueuses et/ou du cuir chevelu ayant subi un vieillissement notamment chronobiologique, pour améliorer le teint de la peau et/ou des muqueuses, notamment l'homogénéiser, en particulier en augmentant l'expression de claudin 5 de la peau, pour améliorer la fermeté et/ou la densité de la peau et/ou des muqueuses et/ou du cuir chevelu, et/ou pour lutter contre la diminution de l'épaisseur de l'épithélium de la peau et/ou des muqueuses et/ou du cuir chevelu, préférentiellement l'épiderme, et/ou pour augmenter l'épaisseur de l'épithélium de la peau et/ou des muqueuses et/ou du cuir chevelu, préférentiellement l'épiderme.

Dans un mode de réalisation particulier, l'utilisation selon la présente invention a pour but d'améliorer le teint de la peau, avantageusement par élimination des rougeurs et/ou par homogénéisation du teint et/ou en lui donnant un aspect lumineux, éclatant, en bonne santé et/ou nourri, un effet bonne mine et/ou un éclat rosé.
En effet, l'éclat du teint traduit en général un état de bonne santé de la peau. De nombreux facteurs intrinsèques ou extrinsèques peuvent provoquer un teint brouillé, inhomogène. Parmi les facteurs affectant l'éclat du teint de la peau, on peut citer le stress, la fatigue, les changements hormonaux, la déshydratation de l'épithélium, préférentiellement l'épiderme, les agents polluants et le vieillissement chronobiologique et photobiologique. Ces facteurs tendent à brouiller le teint, le rendre inhomogène, terne, cireux, voire maladif. L'expression du perlécane et du dystroglycane a un impact sur la voie microvasculaire ce qui permet de mieux nourrir la peau et de mieux la détoxifier donc de lui donner un aspect nourri et en bonne santé. En outre la couleur cutanée est influencée par la microcirculation : en atteignant les microvaisseaux, la lumière entre en contact avec les globules rouges qui absorbent spécifiquement dans le vert. Le rouge est réfléchit à la surface, donnant le teint rosé à la peau et donc un éclat rosé. Ainsi l'augmentation de l'expression de claudin 5 permet d'améliorer l'éclat du teint. L'éclat du teint dépend également du pouvoir réfléchissant de la peau. Ce pouvoir réfléchissant est influencé par la texture de la peau. Une peau ayant une texture plus douce, plus souple aura donc un meilleur respect. La restauration de l'architecture épidermique et l'amélioration de la morphogenèse de l'épithélium, préférentiellement l'épiderme obtenu grâce à la stimulation de l'expression du perlécane et/ou du dystroglycane permettra donc également l'amélioration du teint de la peau. L'amélioration de l'éclat du teint dit « radiance » ou « glow » en anglais peut notamment être mesurée par une méthode instrumentale objective. Cette méthode de mesure in vivo consiste à prendre des photographies hautes résolution en configuration polarisée croisées du visage des volontaires prises à 45° avant et après application du produit testé. Sur la base de ces photographies numériques, une analyse d'image permet d'extraire et de quantifier des paramètres spécifiques (par exemple: L*, a*, b*, C, h°) reliés à la couleur, l'éclat, l'homogénéité, et la texture de la peau.
De même, la brillance dite « gloss » en anglais peut notamment être mesurée selon cette méthode sur la base de photographies hautes résolution en configuration polarisée croisées et polarisée parallèles du visage des volontaires prises à 45° avant et après application du produit testé. Sur la base de ces photographies numériques, une analyse d'image permet d'extraire et de quantifier des paramètres spécifiques reliés à la brillance tels que la brillance spéculaire et la brillance de contraste.

Dans un autre mode de réalisation particulier, l'utilisation selon la présente invention a pour but de prévenir et/ou lutter contre le vieillissement de la peau et/ou des muqueuses et/ou du cuir chevelu notamment chronobiologique ou photobiologique par diminution ou suppression des rides et/ou ridules, en particulier pour les peaux matures et/ou les peaux présentant les premiers signes de vieillissement.
Au sens à présente invention, on entend par « peaux matures », les peaux des femmes ou hommes ayant au minimum 50 ans, avantageusement les peaux des femmes ménopausées.
Au sens de la présente invention, on entend par « peaux présentant les premiers signes de vieillissement », les peaux des femmes ou hommes ayant entre 30 et 40 ans, avantageusement les peaux présentant les premières rides d'expression.

Dans encore un autre mode de réalisation particulier, l'utilisation selon la présente invention a pour but de prévenir et/ou lutter contre la diminution de l'homéostasie de la peau et/ou des muqueuses et/ou du cuir chevelu et/ou l'améliorer, en particulier dans l'épiderme.
L'homéostasie notamment cutanée, et en particulier épidermique, résulte d'un équilibre finement réglé entre les processus de prolifération et de différenciation des cellules de la peau et en particulier des kératinocytes. Ces processus de prolifération et de différenciation participent au renouvellement et/ou à la régénération de la peau et conduisent au maintien d'une épaisseur constante de la peau, et en particulier d'une épaisseur constante de l'épithélium, préférentiellement l'épiderme. Cette homéostasie participe également au maintien des propriétés mécaniques de la peau, des muqueuses et du cuir chevelu.
Mais cette homéostasie cutanée peut être altérée par certains facteurs physiologiques (âge, ménopause, hormones, stress...) et facteurs extrinsèques (agents polluants, ...). Le potentiel régénératif de l'épithélium, notamment l'épiderme devient moins important : les cellules de la couche basale se divisent moins activement, conduisant notamment à un ralentissement et/ou une diminution du renouvellement épidermique. Par conséquent, le renouvellement cellulaire ne compense plus la perte des cellules éliminées en surface, conduisant à une atrophie de l'épithélium, notamment l'épiderme et/ou une diminution de l'épaisseur de la peau et/ou des muqueuses et/ou du cuir chevelu et/ou une perte de densité et/ou de fermeté de la peau et/ou des muqueuses et/ou du cuir chevelu. Ce phénomène peut être accentué par la ménopause. Les déficits hormonaux associés à la ménopause s'accompagnent notamment d'une baisse d'activité métabolique, ce qui pourrait aboutir à une diminution de la prolifération des kératinocytes et une augmentation de la différenciation épidermique. L'utilisation selon la présente invention permet donc de favoriser l'homéostasie afin de maintenir et/ou augmenter l'épaisseur de la peau et/ou des muqueuses et/ou du cuir chevelu et ainsi maintenir et/ou améliorer les propriétés mécaniques de la peau et/ou des muqueuses et/ou du cuir chevelu et/ou améliorer la fermeté et/ou la densité de la peau et/ou des muqueuses et/ou du cuir chevelu, en particulier chez les femmes ménopausées.

L'augmentation de l'épaisseur de l'épithélium, préférentiellement l'épiderme peut notamment être évaluée ex vivo sur modèle de biopsie de peau et/ou muqueuse et/ou cuir chevelu en survie. L'épithélium, préférentiellement l'épiderme est mesuré au début de l'expérience avant l'application du produit à tester et à l'issue de la période de test en présence du produit à tester, par exemple 4 jours préférentiellement 7 jours. L'épaisseur de l'épithélium, préférentiellement l'épiderme est dite augmentée si la mesure après application du produit est égale ou supérieure à 5% à l'issue de la période de test, préférentiellement égale ou supérieure à 10%.

Avantageusement la zone concernée de la peau, en particulier de l'être humain, sur laquelle est appliquée l'extrait de Polygonum bistorta selon l'invention ou une composition cosmétique ou dermatologique le comprenant est choisie parmi le visage, le cou, le décolleté, le buste et /ou les mains, et tout particulièrement les sillons nasogéniens, et/ou la zone périorbitaire, en particulier sur les cernes et les pattes d'oies et/ou le contour des lèvres et/ou le front. Toutefois l'extrait peut également être appliqué sur le corps, et en particulier sur le ventre, les cuisses, les hanches, les fesses et/ou la taille, zones du corps qui peuvent montrer une perte de fermeté et/ou de densité.

Selon l'invention, l'extrait végétal de Polygonum bistorta selon l'invention est utilisé seul ou dans une composition cosmétique ou dermatologique, à une concentration comprise entre 1.10⁻⁴ et 10% en poids, et avantageusement entre 1.10⁻⁴ et 5% et plus particulièrement entre 1.10⁻³ et 3% en poids par rapport au poids de la composition totale. Avantageusement l'extrait de Polygonum bistorta est présent dans une composition cosmétique ou dermatologique selon l'invention en une teneur comprise entre 1.10⁻⁴ à 10% en poids par rapport au poids total de la composition, préférentiellement entre 1.10⁻⁴ et 5% en poids par rapport au poids total de la composition, avantageusement entre 1.10⁻³ et 3% en poids par rapport au poids total la composition, en particulier entre 0,001 et 0,1 % en poids par rapport au poids total la composition.
De manière avantageuse, l'extrait de Polygonum bistorta contient une quantité de resvératrol et/ou de transstilbène (C₁₄H₁₂, MW 180,24 g/mol) et/ou de dérivés de resvératrol notamment sous forme d'oligomères de resvératrol et/ou d'analogues du resvératrol tel que la rhapontine (de formule C₂₁H₂₄O₉, MW 420,14 g/mol), la déoxyrhapontine (C₂₁H₂₄O₈, MW 404,14 g/mol), l'epsilone-viniférine, les acétates, les esters formiques, le 3,4',5-trihydroxystilbène-3-O-beta-D-glucopyranoside (C₂₀H₂₂O₈, MW 390,13 g/mol), et/ou les glucosides du resvératrol, inférieure à 0,001%, préférentiellement inférieure à 0,0001% en poids par rapport au poids total de la composition.
De manière préférentielle, la composition selon l'invention contient une quantité de resvératrol et/ou de transstilbène (C₁₄H₁₂, MW 180,24 g/mol) et/ou de dérivés de resvératrol notamment sous forme d'oligomères de resvératrol et/ou d'analogues du resvératrol tel que la rhapontine (de formule C₂₁H₂₄O₉, MW 420,14 g/mol), la déoxyrhapontine (C₂₁H₂₄O₈, MW 404,14 g/mol), l'epsilone-viniférine, les acétates, les esters formiques et/ou le 3,4',5-trihydroxystilbène-3-O-beta-D-glucopyranoside (C₂₀H₂₂O₈, MW 390,13 g/mol), et/ou les glucosides du resvératrol, inférieure à 0,001%, préférentiellement inférieure à 0,0001% en poids par rapport au poids total de la composition. De manière encore plus préférentielle, la composition selon l'invention ne contient pas de resvératrol et/ou de transstilbène (C₁₄H₁₂, MW 180,24 g/mol) et/ou de dérivés de resvératrol notamment sous forme d'oligomères de resvératrol et/ou d'analogues du resvératrol tel que la rhapontine (de formule C₂₁H₂₄O₉, MW 420,14 g/mol), la déoxyrhapontine (C₂₁H₂₄O₈, MW 404,14 g/mol), l'epsilone-viniférine, les acétates, les esters formiques, et/ou le 3,4',5-trihydroxystilbène-3-O-beta-D-glucopyranoside (C₂₀H₂₂O₈, MW 390,13 g/mol),

Dans un mode de réalisation selon la présente invention, l'extrait de Polygonum bistorta est utilisé pour la fabrication d'une composition dermatologique pour le soin et/ou le traitement dermatologique de la couperose, des télangiectasies, des gerçures, et/ou pour le soin et/ou traitement des pathologies des muqueuses buccales et/ou oculaires, en particulier pour le soin et/ou traitement des pathologies des muqueuses buccales impliquant une perte de fermeté et/ou de densité au niveau de la muqueuse buccale et/ou pour améliorer la structure vasculaire buccale et/ou pour le soin et/ou traitement des pathologies des muqueuses oculaires impliquant une perte de fermeté et/ou de densité au niveau de la muqueuse oculaire et/ou pour améliorer la structure vasculaire oculaire.

Dans un autre mode de réalisation selon la présente invention, l'extrait de Polygonum bistorta selon l'invention est présent dans une composition cosmétique ou dermatologique comprenant un excipient cosmétiquement ou dermatologiquement acceptable.
Cette composition cosmétique ou dermatologique est destinée à une application par voie topique.
Les termes "excipients cosmétiquement ou dermatologiquement acceptable", utilisés ici, signifient que la composition ou les composants de celle-ci sont adaptés à l'utilisation en contact avec la peau et/ou muqueuse humaine et/ou le cuir chevelu humain sans toxicité, incompatibilité, instabilité, réponse allergique, ou leurs équivalents, indue. Les compositions cosmétiques ou dermatologiques selon l'invention contiennent donc un excipient cosmétiquement ou dermatologiquement acceptable en plus de l'extrait selon l'invention. Cet excipient est par exemple au moins un composé choisi dans le groupe consistant en les conservateurs, les émollients, les émulsifiants, les tensioactifs, les hydratants, les épaississants, les conditionneurs, les agents matifiants, les stabilisants, les antioxydants, les agents de texture, les agents de brillance, les agents filmogènes, les solubilisants, les pigments, les colorants, les parfums et les filtres solaires. Ces excipients sont de préférence choisis parmi le groupe consistant en les acides aminés et leurs dérivés, les polyglycérols, les esters, les polymères et dérivés de cellulose, les dérivés de Lanoline, les phospholipides, les lactoferrines, les lactoperoxidases, les stabilisants à base de sucrose, les vitamines E et ses dérivés, les cires naturelles et synthétiques, les huiles végétales, les triglycérides, les insaponifiables, les phytostérols, les esters végétaux, les silicones et ses dérivés, les hydrolysats de protéines, l'huile de Jojoba et ses dérivés, les esters lipo/hydrosolubles, les betaines, les aminoxides, les extraits de plantes, les esters de Saccharose, les dioxydes de Titane, les glycines, et les parabens, et encore de préférence parmi le groupe consistant en le stéareth-2, le stéareth-21, le glycol-15 stéaryle éther, le cétéaryl alcool, le phénoxyéthanol, le méthylparaben, l'éthylparaben, le propylparaben, le butylparaben, le butylène glycol, le caprylyl glycol, les tocophérols naturels, la glycérine, le dihydroxycetyl sodium phosphate, l'isopropyl hydroxycétyl éther, le glycol stéarate, le triisononanoin, l'octyl cocoate, le polyacrylamide, l'isoparaffine, le laureth-7, un carbomer, le propylène glycol, l'hexylène glycol, le glycérol, le bisabolol, une diméthicone, l'hydroxyde de sodium, le PEG 30-dipolyhydroxystérate, les caprique/caprylique triglycérides, le cétéaryl octanoate, le dibutyl adipate, l'huile de pépin de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8, Beeswax, les glycérides d'huile de coeur de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée.

La composition cosmétique selon l'invention peut être choisie parmi une solution, aqueuse ou huileuse, une crème ou un gel aqueux ou un gel huileux, notamment un gel douche, un shampoing ; un lait ; une émulsion, une microémulsion ou une nanoémulsion, notamment huile-dans-eau ou eau-dans-huile ou multiple ou siliconée ; un masque ; un sérum ; une lotion ; un savon liquide ; un pain dermatologique ; une pommade ; une mousse ; un patch ; un produit anhydre, de préférence liquide, pâteux ou solide, par exemple sous forme de poudres de maquillage, de bâtonnet ou de stick, notamment sous forme de rouge à lèvre. De façon avantageuse il s'agit d'une crème ou d'un sérum, en particulier un contour des yeux ou des lèvres.
Les compositions selon l'invention sont plus particulièrement appliquées au niveau du visage, préférentiellement quotidiennement, préférentiellement une à deux fois par jour, préférentiellement le matin et/ou le soir. Avantageusement, les compositions cosmétiques selon l'invention contiennent d'autres ingrédients d'intérêt notamment cosmétique, préférentiellement des agents ayant des propriétés similaires. Préférentiellement il s'agit des ingrédients classiques des compositions anti-âges et/ou améliorant la densité et/ou la fermeté de la peau et/ou des muqueuses et/ou améliorant le teint de la peau et/ou l'homéostasie cutanée notamment ceux choisis parmi les agents de comblement, les agents tenseurs, les agents hydratants, les agents stimulants les molécules de la matrice extracellulaire.
Les compositions cosmétiques selon l'invention peuvent également contenir des ingrédients actifs cosmétiques conduisant à un effet complémentaire ou éventuellement synergique tels que des actifs hydratants, des actifs anti-âges, des actifs anti-radicalaires, les agents protecteurs du facteur de croissance des fibroblastes (FGF), les agents stimulant l'activité et/ou la prolifération des fibroblastes et/ou des eaux thermales. Il peut ainsi s'agir par exemple d'agents de coloration de la peau ou pro-pigmentants, d'inhibiteurs de NO-synthase, d'agents anti-séborrhéiques pour le soin des peaux grasses, d'agents stimulant la synthèse de macromolécules dermiques ou épidermiques, notamment de la matrice extracellulaire et/ou empêchant leur dégradation, pour un effet synergique ou complémentaire, d'agents stimulant la prolifération des fibroblastes ou des kératinocytes et/ou la différenciation des kératinocytes, pour un effet synergique ou complémentaire, d'agents antimicrobiens, d'agent tenseurs, d'agents antipollutions ou anti-radicalaires, d'agents apaisants, calmants ou relaxants, d'agents agissant sur la microcirculation pour améliorer l'éclat du teint, en particulier du visage, pour un effet synergique ou complémentaire, d'agents photoprotecteurs, d'agents cicatrisants, d'agents amincissants, d'agents anti âge pour un effet synergique ou complémentaire ou éventuellement d'agents hydratants et/ou renforçant la barrière épidermique.
Les actifs hydratants, émollients ou humectants peuvent renforcer la fonction barrière et diminuer les pertes insensibles en eau et/ou augmenter la teneur en eau de la peau et/ou des muqueuses ou stimuler l'activité sécrétoire des glandes sébacées et/ou stimuler la synthèse d'aquaporine pour améliorer la circulation de l'eau dans les cellules. On peut citer à titre d'exemple non limitatif les actifs suivants : la sérine, l'urée et ses dérivés, les produits commercialisés sous le nom de Marine Filling sphères™, Advances moisturizing complex™, Hyaluronic Filling Spheres™, vegetal filling spheres™ Osmogelline™, Micropatch™, les alkylcelluloses, les lécithines, les composés à base de sphingoïde, les céramides, les phospholipides, le cholestérol et ses dérivés, les glycosphingolipides, les phytostérols (stigmastérol et béta-sitostérol, campestérol), les acides gras essentiels, le 1-2 diacylglycérol, la 4-chromanone, les triterpènes pentacycliques tels que l'acide ursolique, la vaseline, la lanoline, les sucres en particulier le tréhalose et ses dérivés, le rhamnose, fructose, maltose, lactose, erythritol, le mannitol, le D-xylose et le glucose, l'adénosine et ses dérivés, le sorbitol, les alcools polyhydriques, avantageusement en C2-C6, et de façon encore avantageuse en C3-C6, tels que la glycérine, le propylène glycol, le dipropylène glycol, la diglycérine, la polyglycérine et leur mélange, le glycérol et ses dérivés, le polyacrylate de glycérol, le lactate de sodium, le pentanediol, la serine, les acides lactiques, les AHA, les BHA, le pidolate de sodium, le xylitol, le lactate de sodium, l'ectoine et ses dérivés, le chitosane et ses dérivés, le collagène, le plancton, les dérivés stéroïdiens (dont la DHEA, ses dérivés 7-oxydés et/ou 17-alkylés et les sapogénines), le dihydrojasmonate de méthyle, la vitamine D et ses dérivés, un extrait de Malva sylvestres ou un extrait de Centella asiatica, des homopolymères d'acide acrylique, le beta-glucan et en particulier le sodium carboxymethyl beta-glucane, un dérivé C-glycoside tel que ceux décrits dans la demande WO 02/051828, une huile de rosier muscat, un extrait de micro-algue Prophyridium cruentum enrichi en zinc commercialisé par Vincience sous la dénomination Algualane Zinc™, l'arginine, l'acetyl hexapeptide commercialisé par Lipotech sous le nom Diffuporine™, l'hydrolysat de Viola tricolor commercialisé par Silab sous le nom Aquaphyline™.
L'actif peut également être choisi parmi les agents anti-âges, c'est-à-dire ayant notamment un effet restructurant de la barrière cutanée, les agents prévenant et/ou diminuant la glycation des protéines de la peau en particulier des protéines du derme, telles que le collagène, les actifs stimulant le métabolisme énergétique des cellules et leurs mélanges, un agent à action globale anti-âge, en particulier la niacinamide ou vitamine B3 et dérivés. L'agent ayant un effet restructurant de la barrière cutanée peut être choisi parmi un des extraits de levure comme le Relipidium™ de BASF Beauty Care Solutions France SAS, des sphingosines comme la salicyloyl sphingosine, un mélange de xylitol, de xylityl polyglycoside et de xylitan, des extraits de solanacée comme le Lipidessence™ de BASF Beauty Care Solutions France SAS et leurs mélanges. On peut encore citer notamment les céramides, les composés à base de sphingoïdes, les glycosphingolipides, les phospholipides, le cholestérol et ses dérivés, les phytostérols, les acides gras essentiels, le diacylglycérol, la 4-chromanone et dérivés de chromone et leurs mélanges, vitamine B5 ou pantothenate et dérivés
L'actif stimulant le métabolisme énergétique des cellules peut par exemple être choisi parmi la biotine, un mélange de sels de sodium, de manganèse, de zinc et de magnésium d'acide pyrrolidone carboxylique, un mélange de gluconate de zinc, de cuivre et de magnésium et leurs mélanges.
L'agent anti-séborrhéique dans la composition selon l'invention peut être un inhibiteur de 5alpha-réductase, tel que les rétinoïdes, la sarcosine, les sels de zinc, en particulier le gluconate de zinc, le salicylate de zinc, l'acide azélaique et/ou leurs dérivés, et/ou leurs mélanges et un extrait d'Orthosiphon stamineus commercialisé sous le nom de MAT XS™ bright par BASF Beauty Care Solutions France SAS.
La composition peut également contenir un agent absorbeur de sébum, en particulier un talc et/ou un polymère absorbant, un agent antibactérien notamment ceux décrit dans la demande de brevet FR2863893, et en particulier un extrait de Boldo, un tel extrait étant notamment commercialisé par la Demanderesse sous le nom Betapur™, un agent comédolytique en particulier l'acide rétinoïque et un de ses dérivés tels que isotrétinoine, adapalène et/ou acide 13cis rétinoïque et le péroxyde de benzoyle, un agent antibiotique local, en particulier l'érythromycine et/ou le phosphate de clindamycine et leurs mélanges.
Parmi les actifs stimulant la synthèse des macromolécules du derme ou empêchant leur dégradation, on peut citer ceux qui agissent comme :
- un agent stimulant la synthèse de fibronectine, en particulier un extrait de maïs, un tel extrait étant notamment commercialisé par la Demanderesse sous le nom Deliner™ et le palmitoyl pentapeptide commercialisé par la société SEDERMA sous la dénomination commerciale Matrixil™,
- un agent de protection du facteur de croissance des fibroblastes (FGF2) de la matrice extracellulaire contre sa dégradation et/ou sa dénaturation, notamment un extrait d'Hibiscus abelmoscus tel que décrit dans la demande de brevet au nom de la Demanderesse déposée sous le numéro FR0654316 et/ou un agent de stimulation de croissance des fibroblastes par exemple un extrait de soja fermenté contenant des peptides, connu sous le nom de Phytokine™ commercialisé par la Demanderesse et également décrit dans la demande de brevet EP1119344 B1 (Laboratoires Expanscience), et préférentiellement une combinaison de ces deux extraits;
- un agent stimulant la synthèse de laminine, en particulier un extrait de malt modifié par biotechnologie, un tel extrait étant notamment commercialisé par la Demanderesse sous le nom Basaline™ ;
- un agent stimulant l'expression et/ou l'activité de la Hyaluronane synthase 2 (HAS2) tels que les extraits végétaux décrits dans la demande de brevet FR2 893 252 A1 et en particulier un extrait aqueux de Galanga (Alpinia galanga) ;
- un agent stimulant la synthèse de lysyl oxydase like (LOXL) tel que un extrait de Geophila cordifolia et ceux décrits dans la demande de brevet FR2855968, et en particulier un extrait d'aneth ;
- un agent stimulant la synthèse d'ATP intracellulaire, notamment un extrait d'algue Laminaria digitata ;
- un actif stimulant la synthèse des glycosaminoglycanes, tels que le produit de fermentation du lait ;
- un actif stimulateur de collagène tel que le rétinol et/ou la vitamine C ;
- un actif inhibiteur des métalloprotéinases (MMP) telles que plus particulièrement les MMP 1, 2, 3, 9 tel que les rétinoïdes et dérivés, les oligopeptides et les lipopeptides, les lipoaminoacides, l'extrait de malt commercialisé par BASF Beauty Care Solutions France sous la dénomination commerciale Collalift™, l'extrait hydrolysé de pomme de terre commercialisé sous le nom Extracellium™ par BASF Beauty Care solutions France SAS; le lycopène ; les isoflavones, la quercetine, le kaempferol, l'apigenine.
Les agents stimulant la prolifération des kératinocytes, utilisables dans la composition selon l'invention, comprennent notamment les rétinoïdes tels que le rétinol et ses esters, dont le palmitate de rétinyle et le phloroglucinol. Les agents stimulant la différenciation des kératinocytes comprennent par exemple les minéraux tels que le calcium et les lignanes tels que le sécoisolaricirésinol ainsi que l'extrait d'Achillea millefollium commercialisé sous le nom de Neurobiox™ par BASF Beauty Care Solutions France.
Les agents antimicrobiens susceptibles d'être utilisés dans la composition selon l'invention peuvent notamment être choisis parmi le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, l'octoxyglycérine, l'octanoylglycine, le caprylyl glycol, l'acide 10-hydroxy-2-décanoïque, le farnesol, les phytosphingosines et leurs mélanges.
Parmi les agents tenseurs utilisables dans la composition selon la présente invention, on peut citer notamment les polymères synthétiques, tels que les latex de polyuréthanne ou les latex acryliques ; les polymères d'origine naturelle, notamment les polyholosides sous forme d'amidon ou sous forme de carraghénanes, alginates, agars, gellanes, polymères cellulosiques et pectines; les protéines et hydrolysats de protéines végétales de soja ; les silicates mixtes ; les microparticules de cire ; les particules colloïdales de charge inorganique choisies par exemple parmi la silice, les composites silice-alumine ; ainsi que leurs mélanges.
La composition peut comprendre des agents dits antipollution, en particulier piégeur d'ozone que sont par exemple la vitamine C et ses dérivés dont le glucoside d'ascorbyle; les phénols et polyphénols, en particulier les tannins, l'acide ellagique et l'acide tannique; l'épigallocatéchine et les extraits naturels en contenant, en particulier les extraits de thé vert; les anthocyanes; les acides phénols, les stilbènes; des actifs piégeurs de composés aromatiques mono- ou polycycliques les tannins tels que l'acide ellagique et les dérivés indoles et/ou des actifs piégeurs de métaux lourds tels que l'EDTA, des actifs anti-radicalaires tels que la vitamine E et ses dérivés tels que l'acétate de tocophéryle ; les bioflavonoïdes; la coenzyme Q10 ou ubiquinone.
Comme agents apaisants utilisables dans la composition selon l'invention, on peut citer : les triterpènes pentacycliques, l'acide ursolique et ses sels, l'acide oléanolique et ses sels, l'acide bétulinique et ses sels, les sels de l'acide salicylique et en particulier le salicylate de zinc, le bisabolol, l'allantoïne, les huiles insaturées en oméga 3, la cortisone, l'hydrocortisone, l'indométhacine et la beta méthasone, les actifs anti-inflammatoires, et notamment ceux décrits dans la demande FR2847267, en particulier l'extrait de racine de Pueraria lobata commercialisé sous le nom Inhipase™ par BASF Beauty Care Solutions France SAS, les extraits de Theobroma cacao.
Les ingrédients actifs agissant sur la microcirculation (vasoprotecteurs ou vasodilatateurs) peuvent être choisis parmi les flavonoïdes, les ruscogénines, les nicotinates, les huiles essentielles.
Les ingrédients actifs photoprotecteurs ou filtres UVA et/ou UVB utilisables selon la présente invention sont notamment les agents photoprotecteurs actifs dans l'UV-A et/ou l'UV-B, tels que les dérivés de l'acide para-amino-benzoïque notamment UVINUL P25™ commercialisé par BASF, les dérivés salicyliques en particulier l'homosalate seul ou en association avec des oxydes de titane, les dérivés du dibenzoylméthane, les dérivés cinnamiques, les dérivés de diphénylacrylate, dont Octocrylene vendu notamment sous le nom commercial UVINUL N539™ par BASF, les dérivés de la benzophénone, notamment Benzophenone-1 vendue notamment sous le nom commercial UVINUL 400™ par BASF, les dérivés du benzylidène camphre, les dérivés de benzimidazole, les dérivés de triazine, dont Ethylhexyl triazone vendu notamment sous le nom commercial UVINUL T150™ par BASF, les dérivés de benzotriazole, les dérivés anthranilique, les dérivés d'imidazolines les dérivés de benzalmalonate, les dérivés de 4,4-diarylbutadiène, et leurs mélanges.
Les actifs procurant un effet de bien-être tels que ceux mimant les effets des béta-endorphines pour améliorer la fonction barrière de la peau, tels que ceux cités dans la demande de brevet US 2006069032 ; les actifs stimulants la synthèse des béta-endorphines tels que un extrait de la plante Tephrosia purpurea.
Les actifs amincissants peuvent être notamment choisis parmi : les agents inhibiteurs de la lipoprotéine lipase tels que ceux décrits dans le brevet US2003086949 (Coletica) et en particulier un extrait de liane du Pérou (Uncaria tomentosa); les actifs drainants, notamment l'hesperitine laurate (Flavagrum™), or quercitine caprylate (Flavenger™); les agents inhibiteurs de l'enzyme phosphodiestarase, les agents activateurs de l'adenylate cyclase, l'AMPc et/ou les actifs capable de piéger la spermine et/ou la spermidine. On peut citer à titre d'exemple de ces actifs un extrait de racine de Coleus Forskohlii, un extrait de cecropia obtusa, d'Uva lactuca, la caféine, la forskoline, la théophylline, la théobromine et/ou leurs dérivés, un produit de kappa carraghénanes hydrolysé dénommé Slimexcess™ commercialisé par BASF Beauty Care Solutions France SAS et/ou leurs mélanges.
Dans un mode de réalisation particulier la composition cosmétique selon la présente invention ne contient pas d'agent dépigmentant et/ou antityrosinase et/ou inhibant la mélanogénèse.
De nombreux ingrédients cosmétiquement actifs sont connus par l'homme du métier pour améliorer la santé et/ou l'apparence physique de la peau et/ou les muqueuses et/ou du cuir chevelu. L'homme du métier sait formuler les compositions cosmétiques pour obtenir les meilleurs effets. D'autre part les composés décrits dans la présente invention peuvent avoir un effet de synergie lorsqu'ils sont combinés les uns aux autres. Ces combinaisons sont également couvertes par la présente invention. Le CTFA Cosmetic Ingrédient Handbook, Second Edition (1992) décrit différents ingrédients cosmétiques et pharmaceutiques utilisés couramment dans l'industrie cosmétique, qui sont en particulier adaptés à une utilisation topique. Des exemples de ces classes d'ingrédients comprennent, sans en être limité les composés suivants: abrasif, absorbants, composé à but esthétique tel que les parfums ; les pigments ; les colorants ; les huiles essentielles ;les astringents tels que l'huile de clou de girofle, menthol, camphre, l'huile d'eucalyptus, eugénol, menthyl lactate, distillat d'hamélis ; les agents anti-acné ; les agents anti-floculants ; les agents antimousse ; les agents antimicrobiens tels que iodopropyl butylcarbamate ; les antioxydants tels que l'acide ascorbique; les liants ; les additives biologiques ; les agents tampon ; les agents gonflants ; les agents chélatants ; les additifs ; les agents biocides ; les dénaturants ; les épaississants ; et les vitamines ; les matériaux formant des films ; les polymères ; les agents opacifiants ; les ajusteurs de pH ; les agents réducteurs ; les agents de conditionnement tels que les humectants, et les dérivés ou équivalents de ceux-ci.

Dans encore un autre mode de réalisation particulier de la présente invention, l'extrait de Polygonum bistorta selon l'invention, préférentiellement obtenu par extraction aqueuse est dissout dans un solvant notamment polaire, tel que l'eau, un alcool, un polyol, un glycol, ou un de leurs mélanges, préférentiellement un mélange hydroglycolique, encore préférentiellement contenant un glycol choisi parmi le caprylyl glycol, l'hexylène glycol et leurs mélanges.

De manière particulièrement avantageuse, l'extrait selon l'invention est solubilisé dans une solution aqueuse comprenant de l'hexylène glycol, du caprylyl glycol ou leur mélange, avantageusement de l'hexylène glycol et du caprylyl glycol.
De façon avantageuse la solution aqueuse dans laquelle est solubilisé l'extrait de Polygonum bistorta selon l'invention comprend de l'hexylène glycol, en particulier entre 0,1 et 10 % en poids d'hexylène glycol par rapport au poids total la solution aqueuse, plus particulièrement entre 1 et 5 % en poids d'hexylène glycol par rapport au poids total la solution aqueuse.
De façon particulière la solution aqueuse dans laquelle est solubilisé l'extrait de Polygonum bistorta selon l'invention comprend du caprylyl glycol, en particulier entre 0,01 et 5 % en poids de caprylyl glycol par rapport au poids total la solution aqueuse, plus particulièrement entre 0,1 et 1 % en poids de caprylyl glycol par rapport au poids total la solution aqueuse.
De façon particulièrement avantageuse la solution aqueuse dans laquelle est solubilisé l'extrait de Polygonum bistorta selon l'invention comprend de l'hexylène glycol et du caprylyl glycol, en particulier dans les proportions indiquées ci-dessus.
Avantageusement l'extrait de Polygonum bistorta selon l'invention, préférentiellement obtenu par extraction aqueuse est solubilisé dans la solution aqueuse en une teneur comprise entre 0,1 et 10 % en poids par rapport au poids total de la solution aqueuse, en particulier comprise entre 1 et 5% en poids par rapport au poids total de la solution aqueuse. En particulier cette solution aqueuse comprend de l'hexylène glycol et du caprylyl glycol, avantageusement dans les proportions indiquées ci-dessus pour ces composants.
La solution aqueuse dans laquelle est solubilisé l'extrait selon l'invention peut comprendre un agent épaississant et/ou structurant tel que la gomme de xanthane, avantageusement en une teneur comprise entre 0,01 et 5 % en poids par rapport au poids total la solution aqueuse, en particulier entre 0,1 et 1 % en poids par rapport au poids total de la solution aqueuse.

La présente invention concerne en outre un procédé de soin cosmétique caractérisé en ce qu'elle comprend l'application sur au moins une zone concernée de la peau saine et/ou muqueuse saine et/ou du cuir chevelu sain, en particulier d'un être humain, d'un extrait de Polygonum bistorta ou d'une composition cosmétique comprenant un tel extrait pour stimuler l'expression du perlécane et/ou du dystroglycane, en particulier dans la matrice extracellulaire et/ou dans la membrane basale épithéliale, notamment la jonction dermo-épidermique.
Avantageusement ce procédé de soin cosmétique est pour prévenir et/ou lutter contre le vieillissement de la peau et/ou des muqueuses et/ou du cuir chevelu notamment chronobiologique et/ou photobiologique, pour prévenir et/ou lutter contre la diminution de l'homéostasie de la peau et/ou des muqueuses et/ou du cuir chevelu et/ou pour l'améliorer, en particulier dans l'épiderme, pour renforcer la membrane basale épithéliale de la peau et/ou des muqueuses et/ou du cuir chevelu, préférentiellement la jonction dermo-épidermique, pour améliorer la prolifération et/ou la différenciation des kératinocytes, notamment au niveau épidermique, en particulier lié au vieillissement de la peau et/ou des muqueuses et/ou du cuir chevelu, pour prévenir et/ou lutter contre une diminution de la vascularisation de la peau et/ou des muqueuses et/ou du cuir chevelu et/ou pour l'améliorer, en particulier pour améliorer la structure des capillaires de la peau et/ou des muqueuses et/ou du cuir chevelu notamment cutanés, pour améliorer la morphogenèse de l'épithélium, de la peau et/ou des muqueuses et/ou du cuir chevelu préférentiellement l'épiderme, pour restaurer l'architecture épithéliale de la peau et/ou des muqueuses et/ou du cuir chevelu, préférentiellement épidermique, en particulier des peaux et/ou des muqueuses et/ou du cuir chevelu ayant subi un vieillissement notamment chronobiologique, pour améliorer le teint de la peau et/ou des muqueuses, notamment l'homogénéiser, pour améliorer la fermeté et/ou la densité de la peau et/ou des muqueuses et/ou du cuir chevelu, et/ou pour lutter contre la diminution de l'épaisseur de l'épithélium de la peau et/ou des muqueuses et/ou du cuir chevelu, préférentiellement l'épiderme, et/ou pour augmenter l'épaisseur de l'épithélium de la peau et/ou des muqueuses et/ou du cuir chevelu, préférentiellement l'épiderme.

D'autres buts, caractéristiques et avantages de l'invention apparaîtront clairement à l'homme de l'art suite à la lecture de la description explicative qui fait référence à des exemples qui sont donnés seulement à titre d'illustration et qui ne sauraient en aucune façon limiter la portée de l'invention.
Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.
Ainsi, chaque exemple a une portée générale.
D'autre part, dans les exemples, et sauf indication contraire, la température est exprimée en degré Celsius, et la pression est la pression atmosphérique.

### FIGURES

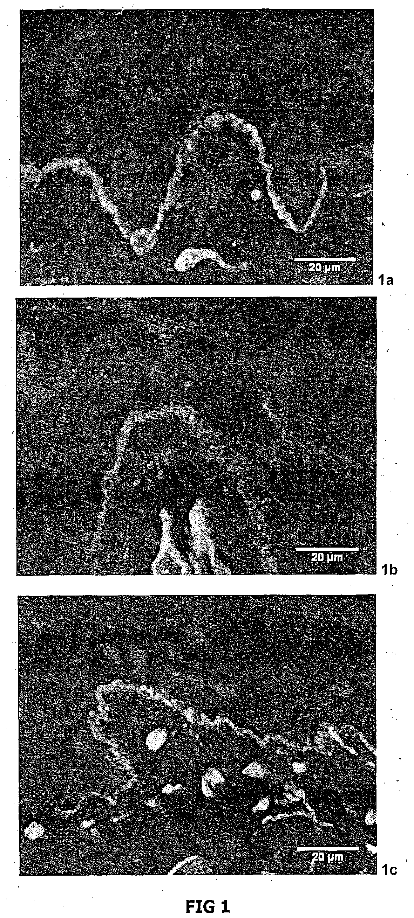

La figure 1 représente l'évolution du taux d'expression du perlécane mesuré dans une biopsie d'un donneur de 50 ans en culture sans traitement (contrôle) ou avec traitement (Extrait à 0,5%) à 0 jour (T0) et 7 jours (T7) de culture selon le test décrit l'exemple 6.

### Exemple 1 : préparation d'extrait de Polygonum bistorta selon l'invention par extraction aqueuse.

**a)** Les racines de Polygonum bistorta sont broyées puis mises à macérer dans l'eau pendant 2 heures à température ambiante c'est-à-dire entre 18 et 25°C, ici à environ 20°C, la teneur en racines de Polygonum bistorta broyées étant de 1% en poids par rapport au poids total plante/eau.
   Les insolubles sont séparés par filtration à 0,45µm et on récupère le liquide qui contient l'extrait aqueux selon l'invention. Cet extrait a été testé à différents dosages dans le milieu de culture final dans les exemples 2, 4, 5 et 6 suivants.
   L'extrait ainsi obtenu ne contient pas de resvératrol, ni de transstilbène (C₁₄H₁₂, MW 180,24 g/mol), ni de rhapontine (de formule C₂₁H₂₄O₉, MW 420,14 g/mol), ni de déoxyrhapontine (C₂₁H₂₄O₈, MW 404,14 g/mol), ni d'epsilone-viniférine, ni de 3,4,5-trihydroxystilbène-3-O-beta-D-glucopyranoside (C₂₀H₂₂O₈, MW 390,13 g/mol).
   Cet extrait peut également être formulé sous la forme d'un ingrédient cosmétique tel que présenté en exemple 7.
**b)** Les racines de Polygonum bistorta sont broyées puis mises à macérer dans l'eau à 1% (p/p), à une température préférentiellement comprise entre 0 et 20°C, préférentiellement à 4°C.
   La durée de macération est avantageusement comprise entre 30min et 24heures, sous agitation, ici 16 heures.
   La solution est centrifugée, préférentiellement pendant 10min à 8000 RPM et le surnageant est récupéré. Le surnageant est ultrafiltré sur filtres à différents seuil de coupure et notamment à 0,22 µM.
   L'extrait ainsi obtenu peut être utilisé directement sous forme liquide. Il a été utilisé à différents dosages dans l'exemple 3.
**c)** Les racines de Polygonum bistorta sont broyées puis mises à macérer dans un mélange eau/butylène glycol à 75%/25% à une température préférentiellement comprise entre 0 et 20°C, ici à 4°C. La durée de macération est avantageusement comprise entre 30min et 24heures, sous agitation, ici 10 heures.

La solution est centrifugée, préférentiellement pendant 10min à 8000 RPM et le surnageant est récupéré. Le surnageant est ultrafiltré sur filtres à différents seuil de coupure et notamment à 0.45µM.

L'extrait ainsi obtenu est ensuite séché notamment sur support de type maltodextrine puis resolubilisé dans de l'eau à 1% (p/p).

### Exemple 2 : Effet d'un extrait de Polygonum bistorta selon l'invention sur l'expression du perlécane dans les kératinocytes.

Un test fluoroimmunoassay (FIA) a été réalisé et consiste à révéler l'antigène d'intérêt, ici, le perlécane, en fluorescence. Cette méthode est semi-quantitative, hautement sensible et reproductible, et présente l'avantage de détecter la protéine d'intérêt sous sa forme native dans son environnement, sans procédé de dénaturation. Les kératinocytes issus d'une biopsie de peau abdominale d'un donneur âgé de 50 ans sont extraits et fixés au fond des puits à une densité de 5000 cellules par puits, et croissent pendant 3 jours dans un milieu complet c'est-à-dire contenant du sérum de veau foetal (SVF), puis 16heures en milieu défini sans SVF. Ils sont ensuite cultivés pendant 48heures soit avec l'extrait obtenu à l'exemple 1a) testé à différents dosages en pourcentage en poids dans le milieu final de culture ou sans l'extrait dit contrôle. Les cellules sont alors lavées en tampon phosphate salin (PBS) avant d'être fixées, perméabilisées, et démasquées. Avant d'appliquer l'anticorps primaire (anti-perlécane) pendant 90 minutes, les cellules sont saturées à la BSA (albumine sérique bovine) à 1% pendant 1 heure. Après des lavages en tampon PBS, l'anticorps secondaire lié au fluorocrome FITC (fluoresceine isothiocyanate) est incubé 2 heures. Les cellules sont alors lavées en tampon PBS, puis solubilisées avec 20 mM d'hydroxyde d'ammonium et 0,5% de triton 100%. La fluorescence est alors lue sur un spectrofluorimètre avec les filtres adéquats. Les résultats sont rassemblés dans le tableau 1 ci-dessous. L'expérience est effectuée 12 fois (n=12). Les valeurs du tableau représentent les valeurs en pourcentage rapportées aux cellules contrôles non traitées. Les valeurs représentent la moyenne de plusieurs expériences sur différents lots d'extraits. « Moy » désigne la moyenne et « EC » désigne l'écart type.

**Tableau 1 : pourcentage d'expression du perlécane dans les kératinocytes en fonction de la dose d'extrait utilisée.**

| | **Moy** | **EC** |
|---|---|---|
| **Contrôle** | 100 | 1 |
| **Extrait de Polygonum bistorta 0,1%** | 157,5 | 24,5 |
| **Extrait de Polygonum bistorta 0,5%** | 266,9 | 40,5 |
| **Extrai**t **de Polygonum bistorta 1%** | 175,3 | 45,1 |

### Conclusions :

L'extrait selon l'invention induit une augmentation significative de l'expression protéique du perlécane dans les kératinocytes. L'extrait selon l'invention induit donc une amélioration de la cohésion structurale de l'épithélium, préférentiellement l'épiderme.

### Exemple 3 : Effet d'un extrait de Polygonum bistorta selon l'invention sur l'expression du perlécane dans les cellules endothéliales.

La technique est la même que dans l'exemple 2 sauf qu'elle est mise en oeuvre sur des cellules endothéliales extraites de biopsie de peau abdominale de donneurs âgés de 50 ans ou 30 ans ou néonatales.

Les résultats sont rassemblés dans les tableaux 2, 2bis, 3 et 4 ci-dessous ; « Moy » désigne la moyenne et « EC » désigne l'écart type.

**Tableau 2 : pourcentage d'expression du perlécane dans les cellules endothéliales d'un donneur de 50 ans en fonction de la dose d'extrait utilisée. n=12 ; l'extrait de Polygonum bistorta est obtenu selon l'exemple 1b) testé à différents dosages en pourcentage en poids dans le milieu final de culture.**

| | **Moy** | **EC** |
|---|---|---|
| **Contrôle** | 100 | 4,2 |
| **Extrait de Polygonum bistorta 0,1%** | 127 | 2,9 |
| **Extrait de Polygonum bistorta 0,5%** | 122 | 7,6 |

**Tableau 2bis : pourcentage d'expression du perlécane dans les cellules endothéliales d'un donneur de 50 ans en fonction de la dose d'extrait utilisée. n=12 ; l'extrait de Polygonum bistorta est obtenu selon l'exemple 1a) testé à différents dosages en pourcentage en poids dans le milieu final de culture.**

| | **Moy** | **EC** |
|---|---|---|
| **Contrôle** | 100 | 25 |
| **Extrait de Polygonum bistorta 0,1%** | 166 | 16 |
| **Extrait de Polygonum bistorta 0,5%** | 200 | 28 |
| **Extrait de Polygonum bistorta 1%** | 209 | 31 |

**Tableau 3 : pourcentage d'expression du perlécane dans les cellules endothéliales d'un donneur de 30 ans en fonction de l'actif utilisé. n=12 ; l'extrait de Polygonum bistorta est obtenu selon l'exemple 1b) testé à différents dosages en pourcentage en poids dans le milieu final de culture.**

| | **Moy** | **EC** |
|---|---|---|
| **Contrôle, non traitées** | 100 | 10 |
| **Extrait aqueux de Polygonum bistorta 0,1%** | 113 | 4 |
| **Extrait aqueux de Polygonum bistorta 0,5%** | 132 | 15 |

**Tableau 4 : pourcentage d'expression du perlécane dans les cellules endothéliales d'un donneur néonatal en fonction de la dose d'extrait utilisée. n=6 ; l'extrait de Polygonum bistorta est obtenu selon l'exemple 1b) testé à différents dosages en pourcentage en poids dans le milieu final de culture.**

| | **Moy** | **EC** |
|---|---|---|
| **Contrôle** | 100 | 6 |
| **Extrait de Polygonum bistorta 0,1%** | 127 | 6 |
| **Extrait de Polygonum bistorta 0,5%** | 145 | 5 |

### Conclusions :

L'extrait selon l'invention a augmenté significativement aux doses testées l'expression protéique du perlécane dans les cellules endothéliales ce qui témoigne de ses propriétés à améliorer la cohésion structurale microvasculaire. Cette augmentation est observée quel que soit l'âge des donneurs. Cet exemple démontre en outre la meilleure efficacité de l'extrait selon l'exemple 1a).

### Exemple 4 : Effet d'un extrait de Polygonum bistorta selon l'invention sur l'expression du dystroglycane dans les kératinocytes.

La technique est la même que dans l'exemple 2 sauf que l'antigène d'intérêt est ici le dystroglycane et l'anticorps utilisé est un anti-dystroglycane.

Les résultats sont rassemblés dans le tableau 5 ci-après ; « Moy » désigne la moyenne et « EC » désigne l'écart type.

**Tableau 5 : pourcentage d'expression du dystroglycane dans les kératinocytes d'un donneur de 50 ans en fonction la dose d'extrait utilisée. n=12 ; l'extrait de Polygonum bistorta est obtenu selon l'exemple 1a) testé à différents dosages en pourcentage en poids dans le milieu final de culture.**

| | **Moy** | **EC** |
|---|---|---|
| **Contrôle** | 100 | 22 |
| **Extrait de Polygonum bistorta 0,1%** | 147 | 3 |
| **Extrait de Polygonum bistorta 0,5%** | 180 | 6 |
| **Extrait de Polygonum bistorta 1%** | 245 | 5 |

### Conclusions:

L'extrait selon l'invention a augmenté significativement aux doses testées l'expression protéique du dystroglycane dans les kératinocytes. L'extrait selon l'invention induit donc une amélioration de la cohésion structurale de l'épithélium, préférentiellement l'épiderme.

### Exemple 5 : Effet d'un extrait de Polygonum bistorta selon l'invention sur l'expression du dystroglycane dans les cellules endothéliales.

La technique est la même que dans l'exemple 2 sauf que l'antigène d'intérêt est ici le dystroglycane et l'anticorps utilisé est un anti-dystroglycane et qu'elle est mise en oeuvre sur des cellules endothéliales et non sur des kératinocytes.

Les résultats sont rassemblés dans les tableaux 6 et 7 ci-après; « Moy » désigne la moyenne et « EC » désigne l'écart type.

**Tableau 6 : pourcentage d'expression du dystroglycane dans les cellules endothéliales d'un donneur de 30 ans en fonction de la dose d'extrait utilisée. n=12 ; l'extrait de Polygonum bistorta est obtenu selon l'exemple 1a) testé à différents dosages en pourcentage en poids dans le milieu final de culture.**

| | **Moy** | **EC** |
|---|---|---|
| **Contrôle, non traitées** | 100 | 41 |
| **Extrait de Polygonum bistorta 0,1%** | 178 | 13 |
| **Extrait de Polygonum bistorta 0,5%** | 217 | 12 |
| **Extrait de Polygonum bistorta 1%** | 390 | 50 |

**Tableau 7 : pourcentage d'expression du dystroglycane dans les cellules endothéliales d'un donneur de 50 ans en fonction de la dose d'extrait utilisée. n=12 ; l'extrait de Polygonum bistorta est obtenu selon l'exemple 1a) testé à différents dosages en pourcentage en poids dans le milieu final de culture.**

| | **Moy** | **EC** |
|---|---|---|
| **Contrôle, non traitées** | 100 | 22 |
| **Extrait de Polygonum bistorta 0,1%** | 186 | 19 |
| **Extrait de Polygonum bistorta 0,5%** | 171 | 12 |
| **Extrait de Polygonum bistorta 1%** | 187 | 17 |

### Conclusions :

L'extrait selon l'invention a augmenté significativement aux doses testées l'expression protéique du dystroglycane dans les cellules endothéliales ce qui témoigne de ses propriétés à améliorer la cohésion structurale microvasculaire.

### Exemple 6 : Evaluation ex vivo de l'extrait aqueux de Polygonum bistorta selon l'invention sur l'expression du perlécane dans la membrane basale épithéliale, notamment la jonction dermo-épidermique.

L'efficacité d'un extrait de Polygonum bistorta est évaluée sur une biopsie de peau d'abdomen d'une femme âgée de 50 ans après immunomarquage du perlécane.

La biopsie a été mise en survie pendant une durée de 7 jours en milieu spécifique contenant l'extrait de Polygonum bistorta obtenu selon l'exemple 1a) à 0,5% en poids dans le milieu de culture ou n'en contenant pas (contrôle). La biopsie est en émersion c'est-à-dire que l'épithélium, préférentiellement l'épiderme n'est pas recouvert de milieu. Les marquages immunofluorescents du perlécane sont observés et mesurés au microscope confocale par analyse d'images semi-quantificative.

La localisation de la fluorescence et l'intensité moyenne de fluorescence traduisant l'expression du perlécane au niveau de la jonction dermo-épidermique sont étudiées et mesurées au début de l'expérience et en fin d'expérience. Lorsque la jonction dermo-épidermique est fragmentée, une moyenne des intensités moyennes a été réalisée. Les résultats sont exprimés en unité arbitraire de fluorescence (UAF). Les mesures ont été prises dès la mise en culture de la biopsie « TO » et à 7 jours de culture « T7 ». L'expérience a été mise en oeuvre une fois (n=1). Les résultats sont visualisés dans la figure 1.
FIG l.a montre la localisation et l'intensité de la fluorescence à T0
FIG 1.b montre la localisation et l'intensité de la fluorescence dans une biopsie à T7 (contrôle)
FIG 1.c montre la localisation et l'intensité de la fluorescence dans la biopsie à T7 c'est-à-dire après 7 jours de traitement avec l'extrait

### Conclusions:

La fluorescence a diminué entre T0 (Fig1.a) et T7 (Fig1.b) au niveau de la jonction-dermoépidemique ce qui démontre qu'il y a une diminution de l'expression du perlecan dans la jonction dermo-épidermique au cours du temps de culture (contrôle). En présence de l'extrait de Polygonum bistorta selon l'invention, l'expression protéique du perlécane est en revanche maintenue au cours du temps comme en témoigne la fluorescence observée à T7 (Fig 1.c).

### Exemple 7 : composition comprenant l'extrait selon la présente invention destinée à être incorporée dans une composition cosmétique (ingrédient cosmétique)

L'extrait de Polygonum bistorta est obtenu selon l'exemple 1a) et est mélangé avec les autres ingrédients de la formulation suivante :

| **Ingrédient** | **% en poids par rapport au poids total la composition** |
|---|---|
| Eau | >50 % |
| extrait aqueux de Polygonum Bistorta (ex la) | 0,5-5 % |
| Hexylène glycol | 1-5 % |
| Caprylyl glycol | 0,1-1 % |
| gomme xanthane | 0,1-1 % |

### Exemple 8 : essais cliniques d'une composition comprenant un extrait selon la présente invention sur l'éclat du teint de la peau et sur l'effet antirides.

La composition de l'exemple 7 incluse dans une crème à 1 % en poids par rapport au poids total de la crème cosmétique a été testée sur 50 sujets caucasiens , 44 individus évalués (la moitié ayant plus de 45 ans et la moitié ayant un âge compris entre 25 et 45 ans) en comparaison avec 50 autres sujets caucasiens utilisant un placebo qui consiste en une crème identique mais ne contenant pas la composition de l'exemple 7. La crème est appliquée 2 fois par jour sur tout le visage pendant 8 semaines. L'évaluation des résultats a été faite à 4 semaines par imagerie c'est-à-dire par analyse d'image à partir de macrophotographies prises par VISIA CR® (Canfield), par un dermatologue qui a évalué l'effet (scorage) et par la réponse des consommateurs à un questionnaire.

Ainsi, un dermatologue a scoré l'éclat du teint et la ride de la patte d'oie selon une échelle prédéfinie.

Le système VISIA CR® (Canfield) est un système de capture d'image digitale in vivo. Une analyse successive des images obtenues a par ailleurs permis de déterminer plusieurs paramètres caractérisant l'efficacité du traitement.

Les paramètres de la texture de la peau ont été évalués par analyse d'images. Une texture peut être décrite comme une distribution des niveaux de gris dans l'image et plus précisément dans les régions d'intérêt définies au préalable sur les images de visage. L'entropie reflète la complexité de la structure de la peau. L'entropie diminue lorsque la peau devient plus régulière donc égale et plus douce.

L'homogénéité de la peau a été mesurée par la mesure du contraste entre les niveaux de gris. Celui-ci est corrélé avec le grain de peau et diminue lorsque le grain de peau devient plus fin.

Les deux paramètres ont été déterminés en utilisant les indicateurs Haralick calculés à partir de la matrice de co-occurence.

L'évolution des rides a été évaluée sur les macrophotographies après un alignement spatial des images entre les différents temps de prise de vue. Une identification des rides est ensuite faite par une projection dans l'espace colorimétrique. Après un seuillage, les rides sont identifiées. Un masque correspondant aux régions d'intérêt (ROIs) est défini. La quantification des pixels correspondant aux rides est faite dans les mêmes régions d'intérêt de manière identique avant l'application (T0), après deux semaines de traitement (T2sem) et après quatre semaines de traitement (T4sem).

La longueur cumulée apparente des rides de la patte d'oie a également été mesurée par addition de la longueur de toutes les rides sur une zone donnée. La longueur est déterminée via la taille d'un pixel (1pixel=0,03mm). La surface apparente des rides de la patte d'oie a également été mesurée par calcul de la surface en mm² du nombre de pixels segmentés (1pixel=0,03mm).

Les résultats sont présentés dans les tableaux 8, 9, 10, 11, 12 et 13 ci-après. « Moy » désigne la moyenne, « SEM » désigne l'erreur type à la moyenne et « VAR » désigne la variation en pourcentage par rapport au contrôle T0. Le seuil de significativité versus T0 est indiqué dans la colonne « significativité » selon le test statistique mentionné sous chaque tableau.

**Tableau 8 : évolution de l'éclat du teint en score visuel moyen effectué par un dermatologue avant l'application (T0), après deux semaines (T2sem) puis quatre semaines (T4sem) d'application**

| | **Moy** | **SEM** | **VAR** | **Significativité*** |
|---|---|---|---|---|
| **Contrôle, non traitées T0** | 1,02 | 0,13 | - | - |
| **T2sem** | 1,25 | 0,12 | 22% | p<0,01 |
| **T4sem** | 1,45 | 0,13 | 42% | p<0,001 |

| | | | | |
|---|---|---|---|---|
| *Test de Wicoxon | | | | |

**Tableau 9 : évolution des rides de la patte d'oie en score visuel moyen effectué par un dermatologue avant l'application (T0), après deux semaines (T2sem) puis quatre semaines (T4sem) d'application**

| | **Moy** | **SEM** | **VAR** | **Significativité*** |
|---|---|---|---|---|
| **Contrôle, non traitées T0** | 2,69 | 0,24 | - | - |
| **T2sem** | 2,36 | 0,21 | -12% | p<0,001 |
| **T4sem** | 2,20 | 0,20 | -18% | p<0,001 |

| | | | | |
|---|---|---|---|---|
| * Test de Wicoxon | | | | |

**Tableau 10 : évolution de la douceur de la peau (entropie) par analyse d'images avant l'application (T0), après deux semaines (T2sem) puis quatre semaines (T4sem) d'application**

| | **Moy** | **SEM** | **VAR** | **Significativité*** |
|---|---|---|---|---|
| **Contrôle, non traitées T0** | 5,33 | 0,03 | - | - |
| **T2sem** | 5,18 | 0,03 | -3% | p<0,001 |
| **T4sem** | 5,16 | 0,03 | -3% | p<0,001 |

| | | | | |
|---|---|---|---|---|
| * Test Anova à un facteur | | | | |

**Tableau 11 : évolution de l'homogénéité de la peau (contraste) par analyse d'images avant l'application (T0), après deux semaines (T2sem) puis quatre semaines (T4sem) d'application**

| | **Moy** | **SEM** | **VAR** | **Significativité*** |
|---|---|---|---|---|
| **Contrôle, non traitées T0** | 2,16 | 0,11 | - | - |
| **T2sem** | 1,70 | 0,10 | -21% | p<0,001 |
| **T4sem** | 1,67 | 0,10 | -23% | p<0,001 |

| | | | | |
|---|---|---|---|---|
| * Test Anova à un facteur | | | | |

**Tableau 12 : évolution de la longueur cumulée apparente des rides de la patte d'oie en mm analyse d'images avant l'application (T0), après deux semaines (T2sem) puis quatre semaines (T4sem) d'application**

| | **Moy** | **SEM** | **VAR** | **Significativité*** |
|---|---|---|---|---|
| **Contrôle, non traitées T0** | 566,81 | 34,74 | - | - |
| **T2sem** | 496,49 | 33,80 | -12% | p<0,0001 |
| **T4sem** | 497,00 | 32,26 | -12% | p<0,0001 |

| | | | | |
|---|---|---|---|---|
| * Test Anova à un facteur | | | | |

**Tableau 13 : évolution de la surface apparente des rides de la patte d'oie par analyse d'images en mm² avant l'application (T0), après deux semaines (T2sem) puis quatre semaines (T4sem) d'application**

| | **Moy** | **SEM** | **VAR** | **Significativité*** |
|---|---|---|---|---|
| **Contrôle, non traitées T0** | 138,17 | 8,44 | - | - |
| **T2sem** | 124,63 | 8,30 | -10% | p<0,0001 |
| **T4sem** | 123,50 | 7,97 | -11% | p<0,0001 |

| | | | | |
|---|---|---|---|---|
| * Test Anova à un facteur | | | | |

### Conclusions :

Les propriétés de l'extrait de Polygonum bistorta selon l'invention ont ainsi été observées in vivo et démontrées. En effet, on déduit du tableau 8 que l'extrait selon l'invention améliore l'éclat du teint dés deux semaines d'application et que cet effet s'améliore encore après quatre semaines d'application. Cet effet est également démontré dans les résultats du tableau 11 qui montrent une amélioration de l'homogénéité de la peau et du grain de peau par diminution du contraste. Par ailleurs, la douceur de la peau a été nettement améliorée (tableau 10). L'extrait selon l'invention diminue par ailleurs les rides comme cela est visible dans les résultats du tableau 9, notamment leur longueur apparente (tableau 12) et leur surface apparente (tableau 13) et ceci de manière significative

### Exemple 9 : mise en évidence de claudin-5 en immunofluorescence sur cellules endothéliales en préconfluence

L'efficacité d'un extrait de Polygonum bistorta est évaluée sur culture en monocouche de cellules endothéliales (Lonza) de biopsie de peau humaine normale d'une femme âgée de 50 ans et mise en évidence après immunofluorescence du claudin-5.

Les cellules sont ensemencées, cultivées sur milieu EGM2 (Lonza) et amplifiées pendant 10jours puis réensemencées sur plaque d'immunohisto (Labteck) à 50 000 cellules par cm2 et mises en présence de l'extrait 1a) pendant 48h à 0,5% dans milieu de culture EGM2 (Lonza) ou en contact du milieu de culture ne contenant pas d'extrait (contrôle).

Les marquages immunofluorescents de la claudin-5 sont observés et mesurés au microscope confocale par analyse d'images semi-quantificative.

La localisation de la fluorescence et l'intensité moyenne de fluorescence traduisant l'expression de la claudin-5 au niveau des cellules endothéliales sont étudiées et mesurées en fin d'expérience. Les résultats sont exprimés en unité arbitraire de fluorescence (UAF) et présentés dans le tableau 14 ci-après ; « Moy » désigne la moyenne et « EC » désigne l'écart type.

**Tableau 14 :**

| | **Moy** | **EC** |
|---|---|---|
| **Contrôle, non traitées** | 100 | 2 |
| **Extrait de Polygonum bistorta 0,5%** | 135 | 4 |

### Conclusions :

Par t-test (p<0,027) il a été démontré que la fluorescence a été augmentée de manière significative en 48h en présence de l'extrait selon l'invention. L'extrait selon l'invention permet donc d'augmenter l'expression protéique de la claudin-5 des cellules épithéliales.

Ceci démontre que l'extrait selon l'invention améliore les jonctions serrées au niveau des cellules endothéliales.

### Exemple 10 : compositions contenant l'extrait de Polygonum bistorta selon l'invention.

On procède selon les méthodes connues de l'homme de l'art pour mélanger ensemble les différentes parties A, B, C, D, E, ou F pour préparer une composition selon la présente invention. Les « produits de l'invention » représentent un extrait de Polygonum bistorta et de préférence obtenu selon l'exemple 1a).

Les produits de l'invention peuvent également se présenter sous une forme de liposomes contenant 5% de lécithine de soja et incorporant une solution de soja quaternisé (600 g en final) obtenus selon le mode de réalisation suivant :
30 g de lécithine de soja, 12 g de solution de soja quaternisé, 1,5 g d'extrait de Polygonum bistorta préparé selon l'exemple 1a) sont introduits dans un pilulier et dilué dans 447 g d'eau pure de laboratoire.

Après agitation magnétique pendant 10 minutes à température ambiante, le mélange est homogénéisé violemment pendant 10 minutes, en obtenant ainsi une solution liposomale dans laquelle les liposomes ont une dimension moyenne pouvant varier entre 100 et 800 nanomètres selon les conditions exactes de l'homogénéisation.

La suspension est ensuite laissée sous agitation douce pendant 1 heure. 90g de butylène glycol, 6g de phenoxyethanol et 6g d'hydroxyéthylcellulose (agent de gélification) sont ensuite ajoutés.

**Formulation cosmétique 10a :**

| | | |
|---|---|---|
| A | Eau | qsp 100 |
| | Butylène Glycol | 2 |
| | Glycérine | 3 |
| | Sodium Dihydroxycétyl Phosphate, Isopropyl Hydroxycétyl Ether | 2 |
| B | Glycol Stéarate SE | 14 |
| | Triisononaoin | 5 |
| | Octyl Cocoate | 6 |
| C | Butylène Glycol, Méthylparaben, Ethylparaben, Propylparaben, pH ajusté à 5,5 | 2 |
| D | Produits de l'invention | 0,01 - 10 % |

**Formulation cosmétique 10b :**

| | | |
|---|---|---|
| A | Eau | qsp 100 |
| | Butylène Glycol | 2 |
| | Glycérine | 3 |
| | Polyacrylamide, Isoparafin, Laureth-7 | 2,8 |
| B | Butylène Glycol, Méthylparaben, Ethylparaben, Propylparaben ; | 2 |
| | Phénoxyéthanol, Méthylparaben, Propylparaben, Butylparaben, Ethylparaben | 2 |
| | Butylène Glycol | 0,5 |
| D | Produits de l'invention | 0,01 - 10 % |

**Formulation cosmétique 10c :**

| | | |
|---|---|---|
| A | Carbomer | 0,50 |
| | Propylène Glycol | 3 |
| | Glycérol | 5 |
| | Eau | qsp 100 |
| B | Octyl Cocoate | 5 |
| | Bisabolol | 0,30 |
| | Diméthicone | 0,30 |
| C | Sodium Hydroxide | 1,60 |
| D | Phénoxyéthanol, Méthylparaben, Propylparaben, Butylparaben, Ethylparaben | 0,50 |
| E | Parfum | 0,30 |
| F | Produits de l'invention | 0,01 - 10 % |

**Formulation dermatologique 10D sous forme d'une pommade**

| | | |
|---|---|---|
| A | Excipients | |
| | Polyéthylène basse densité | 5,5 |
| | Paraffine liquid | qsp 100 |
| B | Produit de l'invention* | 0,001 -0,1 |

| | | |
|---|---|---|
| **L'extrait de Polygonum bistorta est celui décrit en exemple 1a) suivi d'une étape de stérilisation et de séchage.* | | |

## Revendications

1. Utilisation cosmétique par voie topique d'un extrait de Polygonum bistorta obtenu par extraction aqueuse, pour stimuler l'expression du perlécane et/ou du dystroglycane, en particulier dans la matrice extracellulaire et/ou dans la membrane basale épithéliale, notamment la jonction dermo-épidermique, l'extrait de Polygonum bistorta étant obtenu par extraction de la racine de Polygonum bistorta.

2. Utilisation selon la revendication 1, **caractérisée en ce que** l'extrait de Polygonum bistorta est obtenu par extraction avec une solution aqueuse contenant au moins 95% en poids d'eau par rapport au poids total de la solution aqueuse, encore plus avantageusement ne contenant pas de butylène glycol, en particulier ne contenant pas d'alcool.

3. Utilisation selon la revendication 1, **caractérisée en ce que** l'extrait de Polygonum bistorta est obtenu par extraction avec une solution aqueuse ne contenant que de l'eau.

4. Utilisation selon l'une quelconque des revendications 1 à 3, pour stimuler l'expression protéique du perlécane et/ou du dystroglycane, en particulier dans la matrice extracellulaire et/ou dans la membrane basale épithéliale, notamment la jonction dermo-épidermique.

5. Utilisation selon l'une quelconque des revendications 1 à 4, pour prévenir et/ou lutter contre le vieillissement de la peau et/ou des muqueuses et/ou du cuir chevelu notamment chronobiologique et/ou photobiologique, pour prévenir et/ou lutter contre la diminution de l'homéostasie de la peau et/ou des muqueuses et/ou du cuir chevelu et/ou pour l'améliorer, en particulier dans l'épiderme, pour renforcer la membrane basale épithéliale de la peau et/ou des muqueuses et/ou du cuir chevelu, préférentiellement la jonction dermo-épidermique, pour améliorer la prolifération et/ou la différenciation des kératinocytes, notamment au niveau épidermique, en particulier lié au vieillissement de la peau et/ou des muqueuses et/ou du cuir chevelu, pour prévenir et/ou lutter contre une diminution de la vascularisation de la peau et/ou des muqueuses et/ou du cuir chevelu et/ou pour l'améliorer, en particulier pour améliorer la structure des capillaires de la peau et/ou des muqueuses et/ou du cuir chevelu notamment cutanés, pour améliorer la morphogenèse de l'épithélium, de la peau et/ou des muqueuses et/ou du cuir chevelu préférentiellement l'épiderme, pour restaurer l'architecture épithéliale de la peau et/ou des muqueuses et/ou du cuir chevelu, préférentiellement épidermique, en particulier des peaux et/ou des muqueuses et/ou du cuir chevelu ayant subi un vieillissement notamment chronobiologique, pour améliorer le teint de la peau et/ou des muqueuses, notamment l'homogénéiser, pour améliorer la fermeté et/ou la densité de la peau et/ou des muqueuses et/ou du cuir chevelu, et/ou pour lutter contre la diminution de l'épaisseur de l'épithélium de la peau et/ou des muqueuses et/ou du cuir chevelu, préférentiellement l'épiderme, et/ou pour augmenter l'épaisseur de l'épithélium de la peau et/ou des muqueuses et/ou du cuir chevelu, préférentiellement l'épiderme.

6. Utilisation selon la revendication 5 pour améliorer le teint de la peau par élimination des rougeurs et/ou par homogénéisation du teint et/ou en lui donnant un aspect lumineux, éclatant, en bonne santé et/ou nourri, un effet bonne mine et/ou un éclat rosé.

7. Utilisation selon la revendication 5 pour prévenir et/ou lutter contre le vieillissement cutané notamment chronobiologique par diminution ou suppression des rides et/ou ridules, en particulier pour les peaux matures et/ou les peaux présentant les premiers signes de vieillissement.

8. Utilisation selon la revendication 5 pour prévenir et/ou lutter contre la diminution de l'homéostasie de la peau et/ou des muqueuses et/ou du cuir chevelu et/ou pour améliorer l'homéostasie de la peau et/ou des muqueuses et/ou du cuir chevelu, en particulier dans l'épiderme.

9. Utilisation selon la revendication 5 pour augmenter l'épaisseur de l'épithélium de la peau et/ou des muqueuses et/ou du cuir chevelu, préférentiellement l'épiderme, et/ou pour améliorer la fermeté et/ou la densité de la peau et/ou des muqueuses et/ou du cuir chevelu.

10. Utilisation selon l'une quelconque des revendications 1 à 9, **caractérisée en ce que** l'extrait de Polygonum bistorta est appliqué par voie topique sur au moins une zone concernée de la peau saine et/ou d'une muqueuse saine et/ou du cuir chevelu sain, en particulier d'un être humain.

11. Utilisation selon la revendication 10, **caractérisée en ce que** la zone concernée de la peau est choisie parmi le visage, le cou, le décolleté, le buste et /ou les mains, et tout particulièrement les sillons nasogéniens, et/ou la zone périorbitaire, et/ou le contour des lèvres, et/ou du front.

12. Utilisation selon l'une quelconque des revendications 1 à 11, **caractérisée en ce que** l'extrait de Polygonum bistorta est présent dans une composition cosmétique comprenant un excipient cosmétiquement acceptable, avantageusement l'extrait de Polygonum bistorta étant présent en une teneur comprise entre 1.10⁻⁴ et 10 % en poids par rapport au poids total de la composition.

13. Utilisation selon l'une quelconque des revendications 1 à 12, **caractérisée en ce que** l'extrait de Polygonum bistorta est solubilisé dans une solution aqueuse comprenant de l'hexylène glycol, du caprylyl glycol ou leur mélange.

14. Utilisation selon la revendication 13, **caractérisée en ce que** l'extrait de Polygonum bistorta est solubilisé en une teneur comprise entre 0,1 et 10 % en poids par rapport au poids total de la solution aqueuse, en particulier comprise entre 1 et 5% en poids par rapport au poids total de la solution aqueuse.

15. Utilisation selon l'une quelconque des revendications 1 à 14, **caractérisée en ce que** la composition cosmétique est choisie parmi une solution, aqueuse ou huileuse, une crème ou un gel aqueux ou un gel huileux, notamment un gel douche, un shampoing ; un lait ; une émulsion, une microémulsion ou une nanoémulsion, notamment huile-dans-eau ou eau-dans-huile ou multiple ou siliconée ; un masque ; un sérum ; une lotion ; un savon liquide ; un pain dermatologique ; une pommade ; une mousse ; un patch ; un produit anhydre, de préférence liquide, pâteux ou solide, par exemple sous forme de poudres de maquillage, de bâtonnet ou de stick, notamment sous forme de rouge à lèvre.

16. Procédé de soin cosmétique **caractérisé en ce qu'**il comprend l'application sur au moins une zone concernée de la peau saine et/ou muqueuse saine et/ou du cuir chevelu sain d'un extrait de Polygonum bistorta obtenu par extraction aqueuse ou d'une composition cosmétique comprenant un tel extrait pour stimuler l'expression du perlécane et du dystroglycane, en particulier dans la matrice extracellulaire et/ou dans la membrane basale épithéliale, notamment la jonction dermo-épidermique, l'extrait de Polygonum bistorta étant obtenu par extraction de la racine de Polygonum bistorta.

17. Procédé selon la revendication 16 pour prévenir et/ou lutter contre le vieillissement de la peau et/ou des muqueuses et/ou du cuir chevelu notamment chronobiologique et/ou photobiologique, pour prévenir et/ou lutter contre la diminution de l'homéostasie de la peau et/ou des muqueuses et/ou du cuir chevelu et/ou pour l'améliorer, en particulier dans l'épiderme, pour renforcer la membrane basale épithéliale de la peau et/ou des muqueuses et/ou du cuir chevelu, préférentiellement la jonction dermo-épidermique, pour améliorer la prolifération et/ou la différenciation des kératinocytes, notamment au niveau épidermique, en particulier lié au vieillissement de la peau et/ou des muqueuses et/ou du cuir chevelu, pour prévenir et/ou lutter contre une diminution de la vascularisation de la peau et/ou des muqueuses et/ou du cuir chevelu et/ou pour l'améliorer, en particulier pour améliorer la structure des capillaires de la peau et/ou des muqueuses et/ou du cuir chevelu notamment cutanés, pour améliorer la morphogenèse de l'épithélium, de la peau et/ou des muqueuses et/ou du cuir chevelu préférentiellement l'épiderme, pour restaurer l'architecture épithéliale de la peau et/ou des muqueuses et/ou du cuir chevelu, préférentiellement épidermique, en particulier des peaux et/ou des muqueuses et/ou du cuir chevelu ayant subi un vieillissement notamment chronobiologique, pour améliorer le teint de la peau et/ou des muqueuses, notamment l'homogénéiser, pour améliorer la fermeté et/ou la densité de la peau et/ou des muqueuses et/ou du cuir chevelu, et/ou pour lutter contre la diminution de l'épaisseur de l'épithélium de la peau et/ou des muqueuses et/ou du cuir chevelu, préférentiellement l'épiderme, et/ou pour augmenter l'épaisseur de l'épithélium de la peau et/ou des muqueuses et/ou du cuir chevelu, préférentiellement l'épiderme.

18. Extrait de Polygonum bistorta obtenu par extraction aqueuse ou composition dermatologique contenant un extrait de Polygonum bistorta et un excipient dermatologiquement acceptable pour utilisation par voie topique dans le traitement et/ou la prévention de la couperose, des télangiectasies, des gerçures et/ou des pathologies des muqueuses buccales et/ou oculaires, l'extrait de Polygonum bistorta étant obtenu par extraction de la racine de Polygonum bistorta.

19. Extrait pour utilisation selon la revendication 18 **caractérisé en ce qu'**il est tel que défini dans les revendications 2 , 3, 13 ou 14.

## Patentansprüche

1. Topische kosmetische Verwendung eines Schlangen-Knöterich-Extrakts, der durch eine wässrige Extraktion erhalten wird, um die Expression von Perlecan und/oder Dystroglycan insbesondere in der extrazellulären Matrix und/oder in der Epithelbasalmembran, besonders beim Übergang von Dermis zu Epidermis, zu stimulieren, wobei der Schlangen-Knöterich-Extrakt durch eine Extraktion der Wurzel des Schlangen-Knöterichs erhalten wird.

2. Verwendung nach Anspruch 1, **dadurch gekennzeichnet, dass** der Schlangen-Knöterich-Extrakt durch eine Extraktion mit einer wässrigen Lösung erhalten wird, die im Verhältnis zu dem Gesamtgewicht der wässrigen Lösung mindestens 95 Gew.-% Wasser enthält, wobei sie noch vorteilhafter kein Butylenglykol, insbesondere keinen Alkohol enthält.

3. Verwendung nach Anspruch 1, **dadurch gekennzeichnet, dass** der Schlangen-Knöterich-Extrakt durch eine Extraktion mit einer wässrigen Lösung erhalten wird, die nur Wasser enthält.

4. Verwendung nach einem der Ansprüche 1 bis 3, um die Proteinexpression von Perlecan und/oder Dystroglycan insbesondere in der extrazellulären Matrix und/oder in der Epithelbasalmembran, besonders beim Übergang von Dermis zu Epidermis, zu stimulieren.

5. Verwendung nach einem der vorhergehenden Ansprüche 1 bis 4, um die Alterung der Haut und/oder der Schleimhäute und/oder der Kopfhaut, insbesondere die chronobiologische und/oder photobiologische, zu verhindern und/oder zu bekämpfen, um die Verringerung der Homöostase der Haut und/oder der Schleimhäute und/oder der Kopfhaut zu verhindern und/oder zu bekämpfen und/oder um diese zu verbessern, insbesondere in der Epidermis, um die Epithelbasalmembran der Haut und/oder der Schleimhäute und/oder der Kopfhaut, vorzugsweise beim Übergang von Dermis zu Epidermis zu verstärken, um die Proliferation und/oder die Differenzierung der Keratinozyten, besonders auf der Ebene der Epidermis zu verbessern, was insbesondere mit der Alterung der Haut und/oder der Schleimhäute und/oder der Kopfhaut verbunden ist, um eine Verringerung der Gefäßbildung der Haut und/oder der Schleimhäute und/oder der Kopfhaut zu verhindern und/oder zu bekämpfen und/oder um diese zu verbessern, insbesondere um die Struktur der Kapillargefäße der Haut und/oder der Schleimhäute und/oder der Kopfhaut, besonders kutan, zu verbessern, um die Morphogenese des Epithels, der Haut und/oder der Schleimhäute und/oder der Kopfhaut, vorzugsweise der Epidermis, zu verbessern, um die Epithelarchitektur der Haut und/oder der Schleimhäute und/oder der Kopfhaut, vorzugsweise epidermisch, insbesondere der Haut und/oder der Schleimhäute und/oder der Kopfhaut zu restaurieren, die besonders einer chronobiologischen Alterung unterliegen, um den Teint der Haut und/oder der Schleimhäute zu verbessern, ihn besonders zu vereinheitlichen, um die Festigkeit und/oder Dichte der Haut und/oder der Schleimhäute und/oder der Kopfhaut zu verbessern, und/oder um die Verringerung der Dicke des Epithels der Haut und/oder der Schleimhäute und/oder der Kopfhaut, vorzugsweise der Epidermis, zu bekämpfen, und/oder um die Dicke des Epithels der Haut und/oder der Schleimhäute und/oder der Kopfhaut, vorzugsweise der Epidermis, zu erhöhen.

6. Verwendung nach Anspruch 5, um den Teint der Haut durch eine Beseitigung von Rötungen und/oder durch eine Vereinheitlichung des Teints und/oder dadurch zu verbessern, dass ihm ein frischer, strahlender, gesunder und/oder lebendiger Aspekt, ein Effekt für gutes Aussehen und/oder ein rosa Glanz verliehen wird.

7. Verwendung nach Anspruch 5, um die Hautalterung, besonders chronobiologisch, durch eine Verringerung oder Beseitigung von Falten und/oder Fältchen, insbesondere bei reifer Haut und/oder Haut zu verhindern und/oder zu bekämpfen, die erste Zeichen der Alterung aufweist.

8. Verwendung nach Anspruch 5, um die Verringerung der Homöostase der Haut und/oder der Schleimhäute und/oder der Kopfhaut zu verhindern und/oder zu bekämpfen und/oder um die Homöostase der Haut und/oder der Schleimhäute und/oder der Kopfhaut, insbesondere in der Epidermis, zu verbessern.

9. Verwendung nach Anspruch 5, um die Dicke des Epithels der Haut und/oder der Schleimhäute und/oder der Kopfhaut, vorzugsweise der Epidermis, zu erhöhen, und/oder um die Festigkeit und/oder Dichte der Haut und/oder der Schleimhäute und/oder der Kopfhaut zu verbessern.

10. Verwendung nach einem der Ansprüche 1 bis 9, **dadurch gekennzeichnet, dass** der Schlangen-Knöterich-Extrakt topisch auf mindestens einen betroffenen Bereich der gesunden Haut und/oder einer gesunden Schleimhaut und/oder der gesunden Kopfhaut, insbesondere eines Menschen, aufgebracht wird.

11. Verwendung nach Anspruch 10, **dadurch gekennzeichnet, dass** der betroffene Bereich der Haut ausgewählt ist aus dem Gesicht, dem Hals, dem Dekolleté, der Brust und/oder den Händen, und insbesondere den Nasenfalten und/oder dem periorbitalen Bereich und/oder den Lippenkonturen und/oder der Stirn.

12. Verwendung nach einem der Ansprüche 1 bis 11, **dadurch gekennzeichnet, dass** der Schlangen-Knöterich-Extrakt in einer kosmetischen Zusammensetzung, umfassend einen verträglichen kosmetischen Exzipienten, vorliegt, wobei der Schlangen-Knöterich-Extrakt vorteilhafterweise in einem Gehalt vorliegt, der im Verhältnis zu dem Gesamtgewicht der Zusammensetzung zwischen 1x10⁻⁴ und 10 Gew.-% liegt.

13. Verwendung nach einem der Ansprüche 1 bis 12, **dadurch gekennzeichnet, dass** der Schlangen-Knöterich-Extrakt in einer wässrigen Lösung, umfassend Hexylenglykol, Caprylylglykol oder ein Gemisch davon, löslich gemacht wird.

14. Verwendung nach Anspruch 13, **dadurch gekennzeichnet, dass** der Schlangen-Knöterich-Extrakt in einem Gehalt löslich gemacht wird, der im Verhältnis zu dem Gesamtgewicht der wässrigen Lösung zwischen 0,1 und 10 Gew.-% liegt, insbesondere im Verhältnis zu dem Gesamtgewicht der wässrigen Lösung zwischen 1 und 5 Gew.-% liegt.

15. Verwendung nach einem der Ansprüche 1 bis 14, **dadurch gekennzeichnet, dass** die kosmetische Zusammensetzung ausgewählt ist aus einer Lösung, wässrig oder ölig, einer Creme oder einem wässrigen Gel oder einem öligen Gel, besonders einem Duschgel, einem Shampoo, einer Milch, einer Emulsion, einer Mikroemulsion oder einer Nanoemulsion, besonders Öl-in-Wasser oder Wasser-in-Öl oder multipel oder mit Silikon, einer Maske, einem Serum, einer Lotion, einer Flüssigseife, einem Syndet, einer Pomade, einem Schaum, einem Pflaster, einem wasserfreien, vorzugsweise flüssigen, pastösen oder festen Produkt, zum Beispiel in Form von Schminkpulvern, eines Stäbchens oder eines Stifts, besonders in Form eines Lippenstifts.

16. Kosmetisches Pflegeverfahren, das **dadurch gekennzeichnet ist, dass** es das Aufbringen eines Schlangen-Knöterich-Extrakts, der durch eine wässrige Extraktion erhalten wird oder einer kosmetischen Zusammensetzung, umfassend einen solchen Extrakt, zum Stimulieren der Expression von Perlecan und Dystroglycan, insbesondere in der extrazellulären Matrix und/oder in der Epithelbasalmembran, besonders beim Übergang von Dermis zu Epidermis, auf mindestens einen betroffenen Bereich der gesunden Haut und/oder der gesunden Schleimhaut und/oder der gesunden Kopfhaut umfasst, wobei der Schlangen-Knöterich-Extrakt durch eine Extraktion der Wurzel des Schlangen-Knöterichs erhalten wird.

17. Verfahren nach Anspruch 16, um die Alterung der Haut und/oder der Schleimhäute und/oder der Kopfhaut, besonders chronobiologisch und/oder photobiologisch, zu verhindern und/oder zu bekämpfen, um die Verringerung der Homöostase der Haut und/oder der Schleimhäute und/oder der Kopfhaut zu verhindern und/oder zu bekämpfen und/oder um diese zu verbessern, insbesondere in der Epidermis, um die Epithelbasalmembran der Haut und/oder der Schleimhäute und/oder der Kopfhaut, vorzugsweise beim Übergang von Dermis zu Epidermis zu verstärken, um die Proliferation und/oder die Differenzierung der Keratinozyten, besonders auf der Ebene der Epidermis zu verbessern, was insbesondere mit der Alterung der Haut und/oder der Schleimhäute und/oder der Kopfhaut verbunden ist, um eine Verringerung der Gefäßbildung der Haut und/oder der Schleimhäute und/oder der Kopfhaut zu verhindern und/oder zu bekämpfen und/oder um diese zu verbessern, insbesondere um die Struktur der Kapillargefäße der Haut und/oder der Schleimhäute und/oder der Kopfhaut, besonders kutan, zu verbessern, um die Morphogenese des Epithels, der Haut und/oder der Schleimhäute und/oder der Kopfhaut, vorzugsweise der Epidermis, zu verbessern, um die Epithelarchitektur der Haut und/oder der Schleimhäute und/oder der Kopfhaut, vorzugsweise epidermisch, insbesondere der Haut und/oder der Schleimhäute und/oder der Kopfhaut zu restaurieren, die besonders einer chronobiologischen Alterung unterliegen, um den Teint der Haut und/oder der Schleimhäute zu verbessern, ihn besonders zu vereinheitlichen, um die Festigkeit und/oder Dichte der Haut und/oder der Schleimhäute und/oder der Kopfhaut zu verbessern, und/oder um die Verringerung der Dicke des Epithels der Haut und/oder der Schleimhäute und/oder der Kopfhaut, vorzugsweise der Epidermis, zu bekämpfen, und/oder um die Dicke des Epithels der Haut und/oder der Schleimhäute und/oder der Kopfhaut, vorzugsweise der Epidermis, zu erhöhen.

18. Schlangen-Knöterich-Extrakt, der durch eine wässrige Extraktion erhalten wird, oder dermatologische Zusammensetzung, die einen Schlangen-Knöterich-Extrakt und einen dermatologisch verträglichen Exzipienten zur topischen Verwendung bei der Behandlung und/oder Prävention von Folgendem enthält: Kupferrose, Teleangiektasien, Risse und/oder Pathohologien der Mund- und/oder Augenschleimhäute, wobei der Schlangen-Knöterich-Extrakt durch eine Extraktion der Wurzel des Schlangen-Knöterichs erhalten wird.

19. Extrakt zur Verwendung nach Anspruch 18, **dadurch gekennzeichnet, dass** er der Definition in den Ansprüchen 2, 3, 13 oder 14 entspricht.

## Claims

1. A topical cosmetic use of a *Polygonum bistorta* extract obtained by aqueous extraction, for stimulating the expression of perlecan and/or dystroglycan, in particular in the extracellular matrix and/or in the epithelial basement membrane, especially the dermoepidermal junction, the *Polygonum bistorta* extract being obtained by extraction from the *Polygonum bistorta* root.

2. The use as claimed in claim 1, **characterized in that** the *Polygonum bistorta* extract is obtained by extraction with an aqueous solution containing at least 95% by weight of water based on the total weight of the aqueous solution, still more advantageously which does not contain butylene glycol, in particular which does not contain alcohol.

3. The use as claimed in claim 1, **characterized in that** the extract of *Polygonum bistorta* is obtained by extraction with an aqueous solution which contains only water.

4. The use as claimed in any one of claims 1 to 3, for stimulating perlecan and/or dystroglycan protein expression, in particular in the extracellular matrix and/or in the epithelial basement membrane, especially the dermoepidermal junction.

5. The use as claimed in any one of claims 1 to 4, for preventing and/or combating ageing of the skin and/or of the mucous membranes and/or of the scalp, in particular chronobiological and/or photobiological ageing, for preventing and/or combating a decrease in homeostasis of the skin and/or of the mucous membranes and/or of the scalp and/or for improving it, in particular in the epidermis, for reinforcing the epithelial basement membrane of the skin and/or of the mucous membranes and/or of the scalp, preferentially the dermoepidermal junction, for improving keratinocyte proliferation and/or differentiation, especially at the epidermal level, in particular associated with ageing of the skin and/or of the mucous membranes and/or of the scalp, for preventing and/or combating a decrease in vascularization of the skin and/or of the mucous membranes and/or of the scalp and/or for improving it, in particular for improving the structure of the capillaries of the skin and/or of the mucous membranes and/or of the scalp, which are in particular cutaneous, for improving the morphogenesis of the epithelium, of the skin and/or of the mucous membranes and/or of the scalp, preferentially the epidermis, for restoring the epithelial architecture of the skin and/or of the mucous membranes and/or of the scalp, preferentially the epidermal architecture, in particular of skin and/or of mucous membranes and/or of the scalp having undergone ageing, in particular chronobiological ageing, for improving the complexion of the skin and/or of the mucous membranes, especially making it uniform, for improving the firmness and/or the density of the skin and/or of the mucous membranes and/or of the scalp, and/or for combating a decrease in the thickness of the epithelium of the skin and/or of the mucous membranes and/or of the scalp, preferentially the epidermis, and/or for increasing the thickness of the epithelium of the skin and/or of the mucous membranes and/or of the scalp, preferentially the epidermis.

6. The use as claimed in claim 5, for improving the skin complexion by eliminating red patches and/or by making the complexion uniform and/or by giving it a luminous, radiant, healthy and/or nourished appearance, a well-looking effect and/or a pinkish radiance.

7. The use as claimed in claim 5, for preventing and/or combating skin ageing, in particular chronobiological ageing, by decreasing or suppressing wrinkles and/or fine lines, in particular for mature skin and/or skin showing the first signs of ageing.

8. The use as claimed in claim 5, for preventing and/or combating a decrease in the homeostasis of the skin and/or of the mucous membranes and/or of the scalp and/or for improving the homeostasis of the skin and/or of the mucous membranes and/or of the scalp, in particular in the epidermis.

9. The use as claimed in claim 5, for increasing the thickness of the epithelium of the skin and/or of the mucous membranes and/or of the scalp, preferentially the epidermis, and/or for improving the firmness and/or the density of the skin and/or of the mucous membranes and/or of the scalp.

10. The use as claimed in any one of claims 1 to 9, **characterized in that** the *Polygonum bistorta* extract is topically applied to at least one concerned area of healthy skin and/or of a healthy mucous membrane and/or of healthy scalp, in particular of a human being.

11. The use as claimed in claim 10, **characterized in that** the concerned area of the skin is chosen from the face, the neck, the neckline, the bust and/or the hands, and quite particularly the nasal grooves, and/or the periorbital area, and/or the outline of the lips and/or the forehead.

12. The use as claimed in any one of claims 1 to 11, **characterized in that** the *Polygonum bistorta* extract is present in a cosmetic composition comprising a cosmetically acceptable excipient, the *Polygonum bistorta* extract advantageously being present in a content of between 1×10⁻⁴ and 10% by weight relative to the total weight of the composition.

13. The use as claimed in any one of claims 1 to 12, **characterized in that** the *Polygonum bistorta* extract is solubilized in an aqueous solution comprising hexylene glycol, caprylyl glycol or a mixture thereof.

14. The use as claimed in claim 13, **characterized in that** the *Polygonum bistorta* extract is solubilized in a content of between 0.1% and 10% by weight relative to the total weight of the aqueous solution, in particular of between 1% and 5% by weight relative to the total weight of the aqueous solution.

15. The use as claimed in any one of claims 1 to 14, **characterized in that** the cosmetic composition is chosen from an aqueous or oily solution, a cream or an aqueous gel or an oily gel, in particular a shower gel, a shampoo; a milk; an emulsion, a microemulsion or a nanoemulsion, which is in particular oil-in-water or water-in-oil or multiple or silicone-based; a mask; a serum; a lotion; a liquid soap; a dermatological bar; an ointment; a foam; a patch; an anhydrous product, which is preferably liquid, paste or solid, for example in the form of makeup powders, of a wand or of a stick, in particular in the form of a lipstick.

16. A cosmetic care method, **characterized in that** it comprises the application, to at least one concerned area of healthy skin and/or healthy mucous membrane and/or healthy scalp, of a *Polygonum bistorta* extract obtained by aqueous extraction or of a cosmetic composition comprising such an extract for stimulating the expression of perlecan and of dystroglycan, in particular in the extracellular matrix and/or in the epithelial basement membrane, especially the dermoepidermal junction, the *Polygonum bistorta* extract being obtained by extraction from the *Polygonum bistorta* root.

17. The method as claimed in claim 16, for preventing and/or combating ageing of the skin and/or of the mucous membranes and/or of the scalp, in particular chronobiological and/or photobiological ageing, for preventing and/or combating a decrease in homeostasis of the skin and/or of the mucous membranes and/or of the scalp and/or for improving it, in particular in the epidermis, for reinforcing the epithelial basement membrane of the skin and/or of the mucous membranes and/or of the scalp, preferentially the dermoepidermal junction, for improving keratinocyte proliferation and/or differentiation, especially at the epidermal level, in particular associated with ageing of the skin and/or of the mucous membranes and/or of the scalp, for preventing and/or combating a decrease in vascularization of the skin and/or of the mucous membranes and/or of the scalp and/or for improving it, in particular for improving the structure of the capillaries of the skin and/or of the mucous membranes and/or of the scalp, which are in particular cutaneous, for improving the morphogenesis of the epithelium, of the skin and/or of the mucous membranes and/or of the scalp, preferentially the epidermis, for restoring the epithelial architecture of the skin and/or of the mucous membranes and/or of the scalp, preferentially the epidermal architecture, in particular of skin and/or of mucous membranes and/or of the scalp having undergone ageing, in particular chronobiological ageing, for improving the complexion of the skin and/or of the mucous membranes, especially making it uniform, for improving the firmness and/or the density of the skin and/or of the mucous membranes and/or of the scalp, and/or for combating a decrease in the thickness of the epithelium of the skin and/or of the mucous membranes and/or of the scalp, preferentially the epidermis, and/or for increasing the thickness of the epithelium of the skin and/or of the mucous membranes and/or of the scalp, preferentially the epidermis.

18. A *Polygonum bistorta* extract obtained by aqueous extraction or a dermatological composition containing a *Polygonum bistorta* extract and a dermatologically acceptable excipient, for topical use in the treatment and/or prevention of rosacea, telangiectasias, chapping, and/or pathological conditions of the buccal and/or ocular mucous membranes, the *Polygonum bistorta* extract being obtained by extraction from the *Polygonum bistorta* root.

19. The extract as claimed in claim 18, **characterized in that** it is as defined in claims 2, 3, and 13 or 14.
